(19) Europäisches Patentamt
European Patent Office
Office européen des brevets

(11) Veröffentlichungsnummer: **0 409 223 A2**

# (12) EUROPÄISCHE PATENTANMELDUNG

(21) Anmeldenummer: **90113841.2**

(22) Anmeldetag: **19.07.90**

(51) Int. Cl.5: **C07D 513/04**, A61K 31/54, C07D 239/40, //(C07D513/04, 279:00,239:00)

(30) Priorität: **19.07.89 HU 365889**
**19.07.89 HU 365989**

(43) Veröffentlichungstag der Anmeldung:
**23.01.91 Patentblatt 91/04**

(84) Benannte Vertragsstaaten:
**CH DE DK GR LI NL SE**

(71) Anmelder: **EGIS GYOGYSZERGYAR**
**Kereszturi ut 30 - 38**
**H-1106 Budapest(HU)**

(72) Erfinder: **Bozsing, Daniel, Dr.**
**Vézer u. 116**
**H-1141 Budapest(HU)**
Erfinder: **Benko, Pal, Dr.**
**Tartsay V. u. 7**
**H-1126 Budapest(HU)**
Erfinder: **Petocz, Luzja, Dr.**
**Rakoczi tér 2**
**H-1084 Budapest(HU)**
Erfinder: **Szécsey, Maria**
**Kéro u. 6**
**H-1112 Budapest(HU)**
Erfinder: **Tömpe, Péter, Dr.**
**Szaloki u. 29**
**H-1116 Budapest(HU)**
Erfinder: **Gigler, Gabor**
**Lenin krt 95**
**H- Budapest(HU)**
Erfinder: **Gacsalyi, Istvan**
**Baros u. 67**
**H-1201 Budapest(HU)**
Erfinder: **Gyertyan, Istvan**
**Osszefogas st 9**
**H-1165 Budapest(HU)**

(74) Vertreter: **Beszédes, Stephan G., Dr.**
**Patentanwalt, Münchener Strasse 80a**
**D-8060 Dachau(DE)**

(54) **3,4-Di-(hydro)-2H,6H-pyrimido[2,1-b][1,3]thiazin-derivate und 1,2,3,4-Tetra-(hydro)-pyrimidin-2-thione, Verfahren zu ihrer Herstellung und diese enthaltende Arzneimittel.**

(57) Gegenstand der Erfindung sind 3,4-Di-(hydro)-2H,6H-pyrimido[2,1-b][1,3]thiazin-derivate der allgemeinen Formel

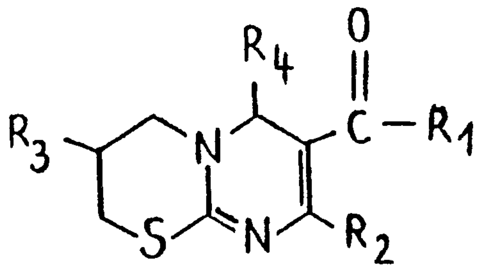

I ,

EP 0 409 223 A2

worin
$R_1$ für einen Alkoxyrest mit 1 bis 6 Kohlenstoffatom(en) oder eine Amino- oder Phenylaminogruppe steht,
$R_2$ einen Alkylrest mit 1 bis 6 Kohlenstoffatom(en) oder einen Phenylrest bedeutet,
$R_3$ ein Wasserstoffatom oder einen Alkylrest mit 1 bis 6 Kohlenstoffatom(en) darstellt
und
$R_4$ für einen Alkylrest mit 1 bis 11 Kohlenstoffatom(en) oder einen, gegebenenfalls durch 1 oder mehrere gleiche oder verschiedene Halogenatom(e), Nitrogruppe(n), Aminogruppe(n), Dialkylaminorest(e) mit 1 bis 6 Kohlenstoffatom(en) in jedem Alkylteil, Alkylrest(e) mit 1 bis 6 Kohlenstoffatom(en), Alkoxyrest(e) mit 1 bis 6 Kohlenstoffatom(en) und/oder Hydroxygruppe(n) substituierten, Phenylrest steht,
sowie ihre Säureadditionssalze.

Ferner sind Gegenstand der Erfindung ein Verfahren zur Herstellung dieser Verbindungen, neue 1,2,3,4-Tetra-(hydro)-pyrimidin-2-thione und ein Verfahren zur Herstellung der letzteren Verbindungen sowie die erstgenannten und/oder letztgenannten Verbindungen enthaltende Arzneimittel und ihre Verwendung.

# 3,4-DI-(HYDRO)-2H,6H-PYRIMIDO[2,1-B][1,3]THIAZIN-DERIVATE UND 1,2,3,4-TETRA-(HYDRO)-PYRIMIDIN-2-THIONE, VERFAHREN ZU IHRER HERSTELLUNG UND DIESE ENTHALTENDE ARZNEIMITTEL

Die Erfindung betrifft neue 3,4-Di-(hydro)-2H,6H-pyrimido[2,1-b][1,3]thiazin-derivate und 1,2,3,4-Tetra-(hydro)-pyrimidin-2-thione, Verfahren zu ihrer Herstellung und diese enthaltende Arzneimittel, insbesondere mit antianginöser und entzündungshemmender Wirkung, wobei die letztgenannten Verbindungen auch wertvolle Zwischenprodukte zur Herstellung der ersteren darstellen.

Di-(hydro)-pyrimido-thiazin-derivate mit entzündungshemmender Wirkung sind in der belgischen Patentschrift 752 863 beschrieben. Diese Verbindungen sind in der 7-Stellung nicht substituiert.

4,5,6-trisubstituierte 1,2,3,4-Tetra-(hydro)-pyrimidin-2-thione, bei welchen sich in der 4-Stellung ein, gegebenenfalls substituierter, Phenylrest befindet, sind bekannt und können beispielsweise nach den in der japanischen Offenlegungsschrift J 59 190 974 oder in der europäischen Offenlegungsschrift EP 202 654 beschriebenen Verfahren hergestellt werden.

Der Erfindung liegt die Aufgabe zugrunde, neue 3,4-Di-(hydro)-2H,6H-pyrimido[2,1-b][1,3]thiazin-derivate und 1,2,3,4-Tetra-(hydro)-pyrimidin-2-thione mit überlegenen pharmakologischen, insbesondere antianginösen und entzündungshemmenden, Wirkungen, Verfahren zur Herstellung derselben und Zwischenprodukte zur Herstellung der erstgenannten Verbindungen sowie die erstgenannten und/oder letztgenannten Verbindungen enthaltende Arzneimittel zu schaffen.

Das Obige wurde überraschenderweise durch die Erfindung erreicht.

Gegenstand der Erfindung sind 3,4-Di-(hydro)-2H,6H-pyrimido[2,1-b][1,3]thiazin-derivate der allgemeinen Formel

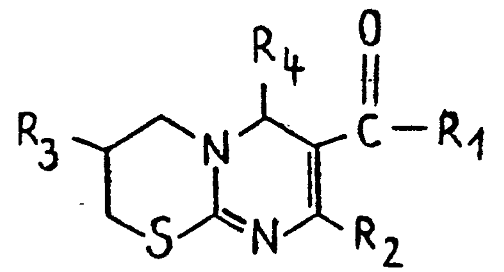

I ,

worin

$R_1$ für einen Alkoxyrest mit 1 bis 6 Kohlenstoffatom(en) oder eine Amino- oder Phenylaminogruppe steht,
$R_2$ einen Alkylrest mit 1 bis 6 Kohlenstoffatom(en) oder einen Phenylrest bedeutet,
$R_3$ ein Wasserstoffatom oder einen Alkylrest mit 1 bis 6 Kohlenstoffatom(en) darstellt und
$R_4$ für einen Alkylrest mit 1 bis 11 Kohlenstoffatom(en) oder einen, gegebenenfalls durch 1 oder mehrere gleiche oder verschiedene Halogenatom(e), Nitrogruppe(n), Aminogruppe(n), Dialkylaminorest(e) mit 1 bis 6 Kohlenstoffatom(en) in jedem Alkylteil, Alkylrest(e) mit 1 bis 6 Kohlenstoffatom(en), Alkoxyrest(e) mit 1 bis 6 Kohlenstoffatom(en) und/oder Hydroxygruppe(n) substituierten, Phenylrest steht,
sowie ihre Säureadditionssalze.

Unter Alkyl- beziehungsweise Alkoxyresten sind gesättigte geradkettige oder verzweigte aliphatische Kohlenwasserstoffreste, beispielsweise Methyl-, Methoxy-, n-Propyl-, n-Propoxy-, tert.-Butyl-, tert.-Butoxy-, n-Hexyl-und n-Hexoxyreste, zu verstehen.

Unter Halogenatomen sind Fluor-, Chlor-, Brom- und Jodatome zu verstehen.

Unter Dialkylaminoresten sind die oben definierten Alkylgruppen enthaltende Reste, zum Beispiel Dimethylamino- und Diäthylaminoreste, zu verstehen.

Vorzugsweise ist beziehungsweise sind der Alkoxyrest, für den $R_1$ stehen kann, der beziehungsweise die Alkylrest(e), für welche[n] $R_2$ , $R_3$ und/oder $R_4$ stehen kann beziehungsweise können und/oder der beziehungsweise die Alkyl- und/oder Alkoxyrest(e) und/oder die Alkylgruppe(n) des beziehungsweise der Dialkylaminoreste[s], durch welche[n] der Phenylrest, für den $R_4$ stehen kann, substituiert sein kann, [ein] solche[r] mit 1 bis 4, insbesondere 1 oder 2, ganz besonders 1, Kohlenstoffatom(en).

Es ist auch bevorzugt, daß das beziehungsweise die Halogenatom(e), durch welche[s] der Phenylrest, für den $R_4$ stehen kann, substituiert sein kann, [ein] Chlor-, Fluor- und/oder Bromatom(e), insbesondere das

beziehungsweise die erstere[n], ist beziehungsweise sind.

Vorzugsweise ist beziehungsweise sind der beziehungsweise die am Phenylrest, für den $R_4$ stehen kann, gegebenenfalls vorliegende(n) Substituent(en) in der beziehungsweise den 3- und/oder 4-Stellung(en) desselben. Bevorzugt ist beziehungsweise sind auch die 2- und/oder 6-Stellung(en). Im Falle des Vorliegens von 3 Substituenten sind auch die 3-, 4- und 5-Stellungen bevorzugt.

Beispiele für substituierte Phenylreste, für die $R_4$ stehen kann, sind 2-(Fluor)-6-(chlor)-phenyl-, 3,4-Di-(chlor)-phenyl-, 3-(Nitro)-phenyl-, 4-(Nitro)-phenyl-, 4-(Methoxy)-phenyl-, 4-(Chlor)-3-(nitro)-phenyl-, 4-(Brom)-phenyl-, 3,4,5-Tri-(methoxy)-phenyl-, 4-(Methyl)-phenyl-, 4-(Dimethylamino)-phenyl-, 2-(Methoxy)-phenyl- und 3-(Hydroxy)-4-(methoxy)-phenylreste.

Bevorzugte erfindungsgemäße 3,4-Di-(hydro)-2H,6H-pyrimido[2,1-b][1,3]thiazin-derivate sind solche, bei welchen

$R_1$ für einen Methoxy- oder Äthoxyrest oder eine Aminogruppe steht,

$R_2$ einen Methyl- oder Phenylrest bedeutet,

$R_3$ Wasserstoff darstellt und

$R_4$ für einen Methylrest oder einen, gegebenenfalls durch 1 oder mehrere gleiche oder verschiedene Methoxyrest(e), Nitrogruppe(n) und/oder Halogenatom(e) substituierten Phenylrest steht,

sowie ihre Säureadditionssalze.

Besonders bevorzugte 3,4-Di-(hydro)-2H,6H-pyrimido[2,1-b][1,3]thiazin-derivate sind die folgenden: Äthyl-[6-(4-dimethyl-aminophenyl)-8-methyl-3,4-dihydro-2H,6H-pyrimido[2,1-b][1,3]thiazin-7-carboxylat], Methyl-[8-methyl-6-(2-methoxy-phenyl)-3,4-dihydro-2H,6H-pyrimido[2,1-b][1,3]thiazin-7-carboxylat], Methyl-[6-(3,4-dichlor-phenyl)-8-methyl-3,4-dihydro-2H,6H-pyrimido[2,1-b][1,3]thiazin-7-carboxylat] und Methyl-[6-(4-chlor-3-methylphenyl)-8-methyl-3,4-dihydro-2H,6H-pyrimido[2,1-b][1,3]thiazin-7-carboxylat] sowie ihre Säureadditionssalze.

Die erfindungsgemäßen 3,4-Di-(hydro)-2H,6H-pyrimido[2,1-b][1,3]thiazin-derivate der allgemeinen Formel I sind als organische Basen zur Bildung von Säureadditionssalzen befähigt. Zweckmäßig sind die erfindungsgemäßen Säureadditionssalze der 3,4-Di-(hydro)-2H,6H-pyrimido[2,1-b][1,3]thiazin-derivate der allgemeinen Formel I solche mit therapeutisch brauchbaren Säuren, vorzugsweise wasserlösliche. Solche können schnell absorbiert werden. Bevorzugte erfindungsgemäße Säureadditionssalze sind Hydrohalogenide, insbesondere Hydrochloride und Hydrobromide, Carbonate, Bicarbonate und Sulfate sowie Acetate, Fumarate, Maleate, Citrate and Ascorbinate.

Gegenstand der Erfindung ist auch ein Verfahren zur Herstellung der erfindungsgemäßen 3,4-Di-(hydro)-2H,6H-pyrimido[2,1-b][1,3]thiazin-derivate, welches dadurch gekennzeichnet ist, daß 4,5,6-trisubstituierte 1,2,3,4-Tetra-(hydro)-pyrimidin-2-thione der allgemeinen Formel

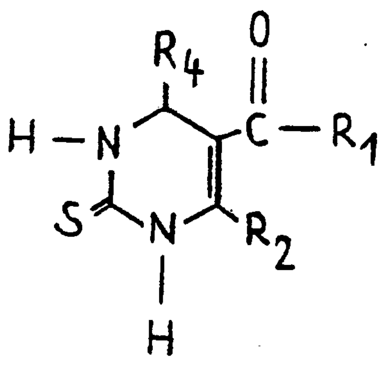

II ,

worin

$R_1$ , $R_2$

und $R_4$ die oben angegebenen Bedeutungen haben, mit Dihalogenalkanen der allgemeinen Formel

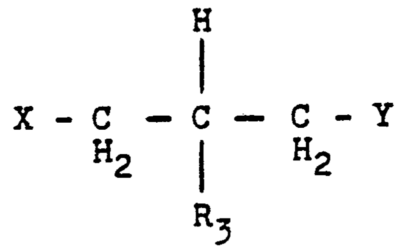

III ,

worin
$R_3$ die oben angegebenen Bedeutungen hat
und
X und Y für Halogenatome stehen,
umgesetzt werden, worauf in an sich bekannter Weise gegebenenfalls die erhaltenen 3,4-Di-(hydro)-2H,6H-pyrimido[2,1-b][1,3]thiazin-derivate der allgemeinen Formel I in Säureadditionssalze überführt werden beziehungsweise gegebenenfalls die erhaltenen Säureadditionssalze der 3,4-Di-(hydro)-2H,6H-pyrimido[2,1-b][1,3]thiazin-derivate der allgemeinen Formel I in die freien 3,4-Di-(hydro)-2H,6H-pyrimido[2,1-b][1,3]-thiazin-derivate der allgemeinen Formel I oder in andere Säureadditionssalze überführt werden.

Die Umsetzung der 4,5,6-trisubstituierten 1,2,3,4-Tetra-(hydro)-pyrimidin-2-thione der allgemeinen Formel II mit den Dihalogenalkanen der allgemeinen Formel III wird vorzugsweise in einem inerten organischen Lösungsmittel oder Lösungsmittelgemisch vorgenommen. So können zweckmäßig aliphatische Alkohole, vorzugsweise Isopropylalkohol und/oder Äthylalkohol, Dialkylamide, vorzugsweise Dimethylformamid, Dialkylsulfoxyde, vorzugsweise Dimethylsulfoxyd, aliphatische Chlorkohlenwasserstoffe, vorzugsweise Chloroform, Tetrachlorkohlenstoff oder Dichlormethan, aromatische Kohlenwasserstoffe, vorzugsweise Benzol, Toluol oder Xylole, aliphatische oder alicyclische Äther, vorzugsweise Diäthyläther, Tetrahydrofuran oder Dioxan, aliphatische Ketone, vorzugsweise Aceton oder Methyläthylketon, oder deren Gemische verwendet werden. Besonders vorteilhaft kann in Dimethylformamid oder in einem Gemisch von Dimethylformamid und Methyläthylketon oder von Dimethylformamid und Aceton gearbeitet werden.

Nach einer vorteilhaften Ausführungsform werden die 4,5,6-trisubstituierten 1,2,3,4-Tetra-(hydro)-pyrimidin-2-thione der allgemeinen Formel II und die Dihalogenalkane der allgemeinen Formel III in äquimolaren Mengen verwendet. Es kann aber auch ein Überschuß der Dihalogenalkane der allgemeinen Formel III eingesetzt werden; dieser beträgt zweckmäßig höchstens 0,5 Mol.

Vorzugsweise wird die Umsetzung der 4,5,6-trisubstituierten 1,2,3,4-Tetra-(hydro)-pyrimidin-2-thione der allgemeinen Formel II mit den Dihalogenalkanen der allgemeinen Formel III in Gegenwart von 1 oder mehr säurebindenden Mittel(n) und/oder zu ihrer Beschleunigung in Gegenwart von 1 oder mehr Katalysator(en) durchgeführt.

Das beziehungsweise die säurebindende(n) Mittel kann beziehungsweise können in äquimolarer Menge oder in einem Überschuß, zweckmäßig bis 1 Mol, bezogen auf die 4,5,6-trisubstituierten 1,2,3,4-Tetra-(hydro)-pyrimidin-2-thione der allgemeinen Formel II oder Dihalogenalkane der allgemeinen Formel III, eingesetzt werden.

Als säurebindende[s] Mittel wird beziehungsweise werden vorzugsweise [ein] Alkalimetallcarbonat(e), beispielsweise Natriumcarbonat und/oder Kaliumcarbonat, Alkalimetallbicarbonat(e), beispielsweise Natrium- und/oder Kaliumbicarbonat, Alkalimetallhydroxyd(e), beispielsweise Natrium- und/oder Kaliumhydroxyd, Erdalkalimetallhydroxyd(e), beispielsweise Calciumhydroxyd, und/oder tertiäre[s] Amin(e), beispielsweise Pyridin, Triäthylamin und/oder [ein] andere[s] Trialkylamin(e), verwendet. Besonders bevorzugt wird Kaliumcarbonat und/oder Natriumcarbonat eingesetzt.

Vorzugsweise wird beziehungsweise werden der beziehungsweise die Katalysator(en) in Mengen von 0,1 bis 0,2 Mol, insbesondere 0,1 Mol, je Mol der 4,5,6-trisubstituierten 1,2,3,4-Tetra-(hydro)-pyrimidin-2-thione der allgemeinen Formel II oder der Dihalogenalkane der allgemeinen Formel III eingesetzt.

Als Katalysator(en) wird beziehungsweise werden vorzugsweise [ein] Alkalimetallhalogenid(e) und/oder Erdalkalimetallhalogenid(e), beispielsweise Kaliumjodid, Kaliumfluorid, Natriumbromid und/oder Calciumchlorid, verwendet. Besonders bevorzugt ist die Verwendung eines Kaliumjodid-Katalysators.

Die Umsetzung der 4,5,6-trisubstituierten 1,2,3,4-Tetra-(hydro)-pyrimidin-2-thione der allgemeinen Formel II mit den Dihalogenalkanen der allgemeinen Formel III kann in Abhängigkeit von der Reaktionsfähigkeit der Ausgangssubstanzen zweckmäßig bei Temperaturen von Raumtemperatur bis zum Siedepunkt des Reaktionsgemisches, vorzugsweise bei Temperaturen von 70 bis 80 °C, durchgeführt werden.

Die Reaktionszeit liegt in Abhängigkeit von der Reaktionsfähigkeit der Ausgangssubstanzen und der

angewandten Temperatur bei 5 bis 36 Stunden.

Das Aufarbeiten des Reaktionsgemisches kann in an sich bekannter Weise durchgeführt werden. Das Isolieren der als Produkte erhaltenen 3,4-Di-(hydro)-2H,6H-pyrimido[2,1-b][1,3]thiazin-derivate wird vcrzugsweise in der Weise vorgenommen, daß die Lösung von den abgeschiedenen anorganischen Salzen durch Filtrieren abgetrennt wird, dann das Lösungsmittel unter Vakuum abdestilliert und der Rückstand mit Wasser oder einem organischen Lösungsmittel oder Lösungsmittelgemisch kristallisiert wird. Erforderlichenfalls wird das Produkt durch bekannte Reinigungsarbeitsgänge, wie Umkristallisieren und/oder chromatographisches Trennen, gereinigt.

Die erfindungsgemäßen 3,4-Di-(hydro)-2H,6H-pyrimido[2,1-b][1,3]thiazin-derivate der allgemeinen Formel I können auch in Form ihrer Säureadditionssalze isoliert werden, oder die als Basen erhaltenen 3,4-Di-(hydro)-2H,6H-pyrimido[2,1-b][1,3]thiazin-derivate der allgemeinen Formel I können in einem folgenden Schritt in der Weise in Salze überführt werden, daß sie in einem inerten Lösungsmittel oder Lösungsmittelgemisch mit den entsprechenden Säuren umgesetzt werden. Aus den Salzen können die Basen erneut freigesetzt werden und in eine andere Salzform überführt werden.

Die im erfindungsgemäßen Verfahren zur Herstellung der erfindungsgemäßen 3,4-Di-(hydro)-2H,6H-pyrimido[2,1-b][1,3]thiazin-derivate der allgemeinen Formel I als Ausgangssubstanzen eingesetzten 4,5,6-trisubstituierten 1,2,3,4-Tetra-(hydro)-pyrimidin-2-thione der allgemeinen Formel III sind handelsübliche bekannte Verbindungen.

Die im erfindungsgemäßen Verfahren zur Herstellung von erfindungsgemäßen 3,4-Di-(hydro)-2H,6H-pyrimido[2,1-b][1,3]thiazin-derivate n der allgemeinen Formel I als Ausgangsubstanzen verwendbaren 4,5,6-trisubstituierten 1,2,3,4-Tetra-(hydro)-pyrimidin-2-thione der allgemeinen Formel II, bei welchen $R_4$ für einen, gegebenenfalls substituierten, Phenylrest steht, sind wie bereits gesagt bekannte Verbindungen.

Die im erfindungsgemäßen Verfahren zur Herstellung von erfindungsgemäßen 3,4-Di-(hydro)-2H,6H-pyrimido[2,1-b][1,3]thiazin-derivaten der allgemeinen Formel I als Ausgangssubstanzen verwendbaren 4,5,6-trisubstituierten 1,2,3,4-Tetra-(hydro)-pyrimidin-2-thione der allgemeinen Formel II, bei welchen $R_4$ für einen Alkylrest mit 1 bis 11 Kohlenstoffatom(en) steht, und ihre Säureadditionssalze sind neu.

Gegenstand der Erfindung sind daher auch 4,5,6-trisubstituierte 1,2,3,4-Tetra-(hydro)-pyrimidin-2-thione der allgemeinen Formel

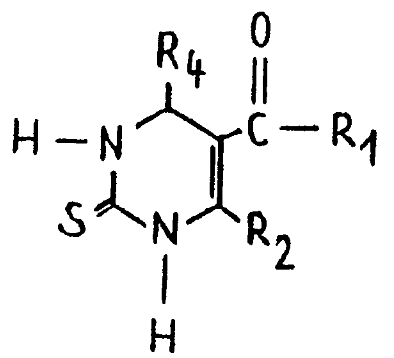

II ,

worin

$R_1$ für einen Alkoxyrest mit 1 bis 6 Kohlenstoffatom(en) oder eine Amino- oder Phenylaminogruppe steht,
$R_2$ einen Alkylrest mit 1 bis 6 Kohlenstoffatom(en) oder einen Phenylrest bedeutet und
$R_4$ für einen Alkylrest mit 1 bis 11 Kohlenstoffatom(en) steht,
sowie ihre Säureadditionssalze,

Außer ihren eigenen überlegenen pharmakologischen Wirkungen sind die Eigenschaften dieser Verbindungen die Ursache für die überlegenen pharmakologischen Wirkungen der aus ihnen herstellbaren erfindungsgemäßen 3,4-Di-(hydro)-2H,6H-pyrimido[2,1-b][1,3]thiazin-derivate der allgemeinen Formel I beziehungsweise Säureadditionssalze derselben.

Gegenstand der Erfindung ist auch ein Verfahren zur Herstellung der erfindungsgemäßen 4,5,6-trisubstituierten 1,2,3,4-Tetra-(hydro)-pyrimidin-2-thione, welches dadurch gekennzeichnet ist, daß Aldehyde der allgemeinen Formel

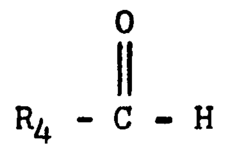

IV ,

worin

$R_4$ die vorstehend angegebene Bedeutung hat, mit $\beta$-Ketocarbonsäurederivaten der allgemeinen Formel

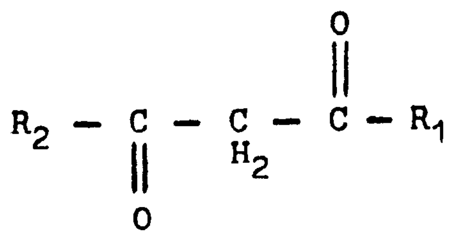

V ,

worin

$R_1$ und $R_2$ die vorstehend angegebenen Bedeutungen haben,

und mit Thioharnstoff umgesetzt werden, worauf in an sich bekannter Weise gegebenenfalls die erhaltenen 1,2,3,4-Tetra-(hydro)-pyrimidin-2-thione der allgemeinen Formel II in Säureadditionssalze überführt werden beziehungsweise gegebenenfalls die erhaltenen Säureadditionssalze der 1,2,3,4-Tetra-(hydro)-pyrimidin-2-thione der allgemeinen Formel II in die freien 1,2,3,4-Tetra-(hydro)-pyrimidin-2-thione der allgemeinen Formel II oder in andere Säureadditionssalze überführt werden.

Die Umsetzung der Aldehyde der allgemeinen Formel IV mit den $\beta$-Ketocarbonsäureestern der allgemeinen Formel V kann in einem inerten organischen Lösungsmittel oder Lösungsmittelgemisch vorgenommen werden. Zweckmäßig können als Lösungsmittel beziehungsweise Lösungsmittelgemische aliphatische Alkohole, beispielsweise Isopropylalkohol und/oder Äthylalkohol, Dialkylamide, vorzugsweise Dimethylformamid, Dialkylsulfoxyde, vorzugsweise Dimethylsulfoxyd, aliphatische Chlorkohlenwasserstoffe, beispielsweise Chloroform, Tetrachlorkohlenstoff oder Dichlormethan, aromatische Kohlenwasserstoffe, beispielsweise Benzol, Toluol oder Xylole, aliphatische oder alicyclische Äther, beispielsweise Diäthyläther, Tetrahydrofuran oder Dioxan, oder deren Gemische eingesetzt werden. Besonders bevorzugt ist die Verwendung von Isopropylalkohol.

Vorteilhaft werden die als Ausgangssubstanzen verwendeten Aldehyde der allgemeinen Formel IV und $\beta$-Ketocarbonsäureester der allgemeinen Formel V in äquimolaren Mengen und der Thioharnstoff in Mengen von 1,0 bis 2,0 Mol, bezogen auf 1 Mol der Aldehyde der allgemeinen Formel IV oder $\beta$-Ketocarbonsäureester der allgemeinen Formel V eingesetzt.

Zur Beschleunigung der Umsetzung kann beziehungsweise können 1 oder mehr Katalysator(en), vorzugsweise Chlorwasserstoffgas in einem wasserfreien organischen Lösungsmittel oder Lösungsmittelgemisch, insbesondere Chlorwasserstoff in Isopropanol, verwendet werden.

Vorzugsweise wird beziehungsweise werden der beziehungsweise die Katalysator(en) in Mengen von 1 bis 7 Mol, insbesondere 1,3 bis 1,35 Mol, je Mol der Aldehyde der allgemeinen Formel IV eingesetzt.

Zweckmäßig wird die Umsetzung bei Temperaturen von 10 bis 50°C, vorzugsweise bei Raumtemperatur, durchgeführt.

Die Reaktionszeit liegt in Abhängigkeit von der Reaktionsfähigkeit der Ausgangssubstanzen bei 3 bis 35 Stunden.

Das Aufarbeiten des Reaktionsgemisches kann in an sich bekannter Weise durchgeführt werden. Das Isolieren der als Produkte erhaltenen 4,5,6-trisubstituierten 1,2,3,4-Tetra-(hydro)-pyrimidin-2-thione der allgemeinen Formel II kann durch einfaches Filtrieren oder durch nach dem Ab dampfen des Lösungsmittels erfolgendes Kristallisieren des Rückstandes mit Wasser, gegebenenfalls mit einem organischen Lösungsmittel, und Filtrieren der so erhaltenen Suspension vorgenommen werden.

Die als Ausgangssubstanzen eingesetzten Aldehyde der allgemeinen Formel IV und $\beta$-Ketocarbonsäureester sind bekannte handelsübliche Verbindungen.

Gegenstand der Erfindung sind auch Arzneimittel, welche durch einen Gehalt an 1 oder mehr erfindungsgemäßen 3,4-Di-(hydro)-2H,6H-pyrimido[2,1-b][1,3]thiazin-derivaten und/oder 4,5,6-trisubstituierten 1,2,3,4-Tetra-(hydro)-pyrimidin-2-thionen, gegebenenfalls zusammen mit 1 oder mehr inerten, festen und/oder flüssigen Träger- und/oder Hilfsstoff(en), gekennzeichnet sind.

Gegenstand der Erfindung ist auch die Verwendung dieser Verbindungen zur Herstellung von Arzneimitteln, insbesondere mit antianginöser und/oder entzündungshemmender Wirkung.

Die erfindungsgemäßen 3,4-Di-(hydro)-2H,6H-pyrimido[2,1-b][1,3]thiazin-derivate haben nämlich wertvolle pharmakologische, insbesondere entzündungshemmende und antianginöse Wirkungen. Diese Wirkungen sind durch eine Wirkung auf das Zentralnervensystem (wie tranquillant-sedative, antidepressive und/oder krampfhemmende Wirkungen) und eine diuretische Wirkung ergänzt, was von Vorteil ist. Wenn nämlich berücksichtigt wird, daß bei Anginaerkrankungen oftmals auslösende Faktoren nervösen Ursprunges eine Rolle spielen beziehungsweise bei Patienten auftreten, bei denen sich bereits auch eine krankhafte Wasserretention gebildet hat, dann stellt die beruhigende oder stimmungsverbessernde Wirkung, gepaart mit einer diureti schen Wirkung, eine komplexere Therapie dar. Bei einzelnen erfindungsgemäßen 3,4-Di-(hydro)-2H,6H-pyrimido[2,1-b][1,3]thiazin-derivaten sind die obigen Wirkungen auch noch durch eine schwache positiv inotrope Wirkung beziehungsweise die Salzsäuresekretion hemmende Wirkung ergänzt.

Auch die 4,5,6-trisubstituierten 1,2,3,4-Tetra-(hydro)-pyrimidin-2-thione haben eine antianginöse Wirkung, welche von einer schmerzlindernden Wirkung begleitet wird.

Die pharmakologischen Untersuchungen der Verbindungen erfolgten mittels aus dem Schrifttum bekannter Verfahrensweisen.

a) Untersuchung der akuten Toxizität von 3,4-Di-(hydro)-2H,6H-pyrimido[2,1-b][1,3]thiazin-derivaten der allgemeinen Formel I an Mäusen

Verfahrensweise

Die Untersuchungen erfolgten an 18 bis 22 g schweren weißen CFLP-Mäusen beiderlei Geschlechtes und zwar je Dosis an 10 Tieren. Die Substanzen wurden in einer Dosis von 20 ml/kg peroral verabreicht. Nach der Behandlung erfolgte eine 14-tägige Beobachtung. Die Tiere wurden in Mäusekisten aus Kunststoff auf einer Streu aus Holzspänen in einem Raum von Raumtemperatur gehalten. Leitungswasser und Standard-Nahrung für Mäuse konnten nach Belieben verzehrt werden. Die Toxizitätsangaben wurden nach der Litchfield-Wilcoxon-Verfahrensweise bestimmt (Litchfield, J. T., Wilcoxon, F. W.: J. Pharmacol, Exp. Ther., 96 [1949], 99).

Die Ergebnisse sind in der folgenden Tabelle I zusammengestellt.

Tabelle I

| Untersuchte Verbindung: Bezeichnung | Untersuchte Verbindung: Beispiel Nr. | Peroraler $LD_{50}$-Wert in mg/kg |
|---|---|---|
| Äthyl-(6-phenyl-8-methyl-3,4-dihydro-2H,6H--pyrimido[2,1-b][1,3]thiazin-7-carboxylat) | 1 | 580 |
| Äthyl-[6-(2-fluor-6-chlorphenyl)-8-methyl--3,4-dihydro-2H,6H-pyrimido[2,1-b][1,3]thiazin--7-carboxylat] | 2 | 170 |
| Äthyl-[6-(3,4-dichlorphenyl)-8-methyl-3,4-dihydro-2H,6H-pyrimido[2,1-b][1,3]thiazin-7--carboxylat] | 3 | 1 000 |
| Methyl-[6-(3-nitrophenyl)-8-methyl-3,4-dihydro--2H,6H-pyrimido-[2,1-b][1,3]thiazin-7-carboxylat] | 4 | 370 |
| 6-Phenyl-8-methyl-3,4-dihydro-2H,6H-pyrimido--[2,1-b][1,3]thiazin-7-carbonsäureanilid | 5 | 700 |

Fortsetzung der Tabelle I

| Untersuchte Verbindung | | Peroraler $LD_{50}$-Wert in mg/kg |
|---|---|---|
| Bezeichnung | Beispiel Nr. | |
| 6-(2-Fluor-6-chlorphenyl)-8-methyl-3,4-dihydro-2H,6H-pyrimido[2,1-b][1,3]thiazin-7-carbonsäure-anilid | 6 | > 1 000 |
| Äthyl-[8-methyl-6-(4-methoxy-phenyl)-3,4-dihydro-2H,6H-pyrimido[2,1-b][1,3]thiazin-7-carboxylat] | 7 | 600 |
| Methyl-[6-(4-chlor-3-nitrophenyl)-8-methyl-3,4-dihydro-2H,6H-pyrimido[2,1-b][1,3]thiazin-7-carboxylat] | 8 | 700 |
| Methyl-[3,8-dimethyl-6-(4-chlor-3-nitrophenyl)-3,4-dihydro-2H,6H-pyrimido[2,1-b][1,3]thiazin-7-carboxylat] | 9 | > 1 000 |
| Methyl-[3,8-dimethyl-6-(3-nitrophenyl)-3,4-dihydro-2H,6H-pyrimido[2,1-b][1,3]thiazin-7-carboxylat] | 10 | > 1 000 |

Fortsetzung der Tabelle I

| Untersuchte Verbindung: Bezeichnung | Untersuchte Verbindung: Beispiel Nr. | Peroraler $LD_{50}$-Wert in mg/kg |
|---|---|---|
| Äthyl-6-(4-bromphenyl)-8-methyl-3,4-dihydro-2H,6H-pyrimido[2,1-b][1,3]thiazin-7-carboxylat | 11 | > 1 000 |
| Äthyl-[8-phenyl-6-(2-fluor-6-chlorphenyl)-3,4-dihydro-2H,6H-pyrimido[2,1-b][1,3]thiazin-7-carboxylat] | 12 | > 1 000 |
| Äthyl-[8-phenyl-6-(4-nitrophenyl)-3,4-dihydro-2H,6H-pyrimido[2,1-b][1,3]thiazin-7-carboxylat] | 13 | 1 000 |
| Methyl-(6,8-dimethyl-3,4-dihydro-2H,6H-pyrimido[2,1-b][1,3]thiazin-7-carboxylat) | 14 | 400 |
| Methyl-[8-methyl-6-(4-methoxyphenyl)-3,4-dihydro-2H,6H-pyrimido[2,1-b][1,3]thiazin-7-carboxylat] | 15 | > 1 000 |

Fortsetzung der Tabelle I

| Untersuchte Verbindung | | Peroraler $LD_{50}$-Wert in mg/kg |
|---|---|---|
| Bezeichnung | Beispiel Nr. | |
| Methyl-[6-(3,4-dichlorphenyl)-8-methyl-3,4-dihydro-2H,6H-pyrimido[2,1-b][1,3]thiazin-7-carboxylat] | 16 | 1 000 |
| Methyl-[8-methyl-6-(3,4,5-trimethoxyphenyl)-3,4-dihydro-2H,6H-pyrimido[2,1-b][1,3]thiazin-7-carboxylat] | 18 | > 1 000 |
| Äthyl-[6-(4-dimethylamino-phenyl)-8-methyl-3,4-dihydro-2H,6H-pyrimido[2,1-b][1,3]thiazin-7-carboxylat] | 22 | 700 |
| Indometacin | Vergleichssubstanz B | 22,5 |

**Fortsetzung der Tabelle I**

| Untersuchte Verbindung | | Peroraler $LD_{50}$-Wert in mg/kg |
|---|---|---|
| Bezeichnung | Beispiel Nr. | |
| Phenybutazon | Vergleichs-substanz C | 1 000 |
| Acetylsalicylsäure | Vergleichs-substanz D | 1 350 |
| Paracetamol | Vergleichs-substanz E | 1 180 |
| Meprobamat | Vergleichs-substanz F | 1 100 |

Untersuchung der antianginösen Wirkung von 3,4-Di-(hydro)-2H,6H-pyrimido[2,1-b][1,3]thiazin-derivaten der allgemeinen Formel I und 4,5,6-trisubstituierten 1,2,3,4-Tetra-(hydro)-pyrimidin-2-thionen der allgemeinen Formel II an Ratten

Verfahrensweise

Die Untersuchungen wurden an 180 bis 220 g schweren Ratten durchgeführt. Die Tiere wurden mit Chloralose-urethan narkotisiert, und es wurde mit Hilfe von Nadelelektroden in der II. Standardableitung das EKG registriert. Die Untersuchung der antianginösen Wirkung erfolgte mittels einer modifizierten Nieschultz-Verfahrensweise (Nieschultz, E., Popendiker, K., Hoffmann, I.: Arzneimittelforschung, 5 [1955], 680). Die Koronarinsuffizienz wurde im Versuch mit Vasopressin (1 IE/kg) intravenös hervorgerufen. Es wurde die Größe der T-Zacke vor und nach der Verabreichung des Vasopressines in der Blindversuchsgruppe und in der behandelten Gruppe gemessen. Die untersuchten Substanzen wurden 2 Minuten vor Verabreichung des Vasopressines intravenös verabreicht.

Die Ergebnisse sind in der folgenden Tabelle II zusammengestellt.

Tabelle II

| Untersuchte Verbindung Bezeichnung | Beispiel Nr. | Bei einer intravenösen Dosis von 2 mg/kg beobachtete Wirkung | Intravenöser $ED_{50}$ - Wert in mg/kg |
|---|---|---|---|
| Methyl-[6-(4-chlor-3-nitrophenyl)-8-methyl-3,4-dihydro-2H,6H-pyrimido[2,1-b][1,3]thiazin-7-carboxylat] | 8 | -71 | 1,21 |
| Äthyl-[8-methyl-6-(4-methoxy-phenyl)-3,4-dihydro-2H,6H-pyrimido[2,1-b][1,3]thiazin-7-carboxylat] | 7 | -51 | 1,87 |
| Äthyl-[8-phenyl-6-(4-methoxyphenyl)-3,4-dihydro-2H,6H-pyrimido[2,1-b][1,3]thiazin-7-carboxylat] | 19 | -51 | 1,45 |
| 6-(2-Fluor-6-chlorphenyl)-8-methyl-3,4-dihydro-2H,6H-pyrimido[2,1-b][1,3]thiazin-7-carbonsäure-anilid | 6 | -48 | |

Fortsetzung der Tabelle II

| Untersuchte Verbindung Bezeichnung | Beispiel Nr. | Bei einer intravenösen Dosis von 2 mg/kg beobachtete Wirkung | Intravenöser $ED_{50}$ - Wert in mg/kg |
|---|---|---|---|
| Äthyl-[8-phenyl-6-(2-fluor-6-chlorphenyl)-3,4-dihydro-2H,6H-pyrimido[2,1-b][1,3]thiazin-7-carboxylat] | 12 | -40 | |
| Äthyl-[6-(2-fluor-6-chlorphenyl)-8-methyl-3,4-dihydro-2H,6H-pyrimido[2,1-b][1,3]thiazin-7-carboxylat] | 2 | -30 | |
| Methyl-[8-methyl-6-(4-methoxyphenyl)-3,4-dihydro-2H,6H-pyrimido[2,1-b][1,3]thiazin-7-carboxylat] | 15 | -40 | |
| Methyl-[6-(3,4-dichlorphenyl)-8-methyl-3,4-dihydro-2H,6H-pyrimido[2,1-b][1,3]thiazin-7-carboxylat] | 16 | -57 | etwa 0,81 |

Fortsetzung der Tabelle II

| Untersuchte Verbindung Bezeichnung | Beispiel Nr. | Bei einer intravenösen Dosis von 2 mg/kg beobachtete Wirkung | Intravenöser $ED_{50}$ - Wert in mg/kg |
|---|---|---|---|
| Methyl-(4,6-dimethyl-1,2,3,4-tetrahydro-2-pyrimidinthion-5-carboxylat) | 30 | -43 | |
| Methyl-(4-äthyl-6-methyl-1,2,3,4-tetrahydro-2-pyrimidinthion-5-carboxylat) | 31 | -30 | |
| 4-Undecyl-6-methyl-1,2,3,4-tetrahydro-2-pyrimidinthion-5-carbonsäure-anilid | 36 | -32 | |
| Prenylamin | Vergleichssubstanz A | -32 | 6,5 |

Aus der obigen Tabelle II geht die Überlegenheit der erfindungsgemäßen Verbindungen gegenüber der Vergleichssubstanz hervor. Die wirksamsten Verbindungen waren 3- bis 5-mal so wirksam wie das Prenylamin.

b) Untersuchung der akuten entzündungshemmenden Wirkung von 3,4-Di-(hydro)-2H,6H-pyrimido[2,1-b][1,3]-thiazin-derivaten

Carraghenin-Ödem an Ratten

Verfahrensweise

In die plantare Hinterbeinoberfläche von 150 bis 180 g schweren Ratten wurde 0,1 ml einer 1 gew.-%-igen Carraghenin-Lösung gespritzt. Die 12 Stunden hungern gelassenen Tiere (Wasser stand frei zur Verfügung) wurden 1 Stunde vor der Behandlung mit der zu untersuchenden Substanz mit Leitungswasser in einer Dosis von 30 ml/kg hydratiert. Die Tiere wurden peroral mit der zu untersuchenden Substanz beziehungsweise die Blindversuchstiere mit dem Träger in einem Volumen von 10 ml/kg behandelt, dann wurde nach Ablauf von 1 Stunde das entzündungserregende beziehungsweise irritierende Mittel verabreicht. Der Umfang der Beine wurde vor und 3 Stunden nach Verabreichung des entzündungserregenden beziehungsweise irritierenden Mittels in einem Plethysmometer bestimmt, indem die sich aus der Umfangsänderung ergebende Flüssigkeitsbewegung an der Millimeterskala abgelesen wurde. Die Veränderung des Beinumfanges der behandelten Gruppe wurde mit der der Blindversuchsgruppe verglichen. Die 30%-ige Hemmdosis ($ID_{30}$-Wert) wurde mit Hilfe einer Regressionsgeraden bestimmt.

Die Ergebnisse sind in der folgenden Tabelle III zusammengestellt.

Tabelle III

| Untersuchte Verbindung: Bezeichnung | Untersuchte Verbindung: Beispiel Nr. | Peroraler $LD_{50}$-Wert in mg/kg | $ID_{30}$-Wert in mg/kg | Therapeutischer Index $\left(\frac{LD_{50}\text{-Wert}}{ID_{30}\text{-Wert}}\right)$ |
|---|---|---|---|---|
| Methyl-[8-methyl-6-(2-methoxy-phenyl)-3,4-dihydro-2H,6H-pyrimido[2.1-b][1,3]thiazin-7-carboxylat] | 25 | 100 bis 500 | 10 | 10 bis 50 |
| Methyl-(6,8-dimethyl-3,4-dihydro-2H,6H-pyrimido[2,1-b][1,3]thiazin-7-carboxylat) | 14 | 400 | 10 | 40 |
| Methyl-[8-methyl-6-(4-methoxy-phenyl)-3,4-dihydro-2H,6H-pyrimido[2,1-b][1,3]thiazin-7-carboxylat] | 15 | $>$ 1 000 | 70 | $>$ 14,3 |

Fortsetzung der Tabelle III

| Untersuchte Verbindung Bezeichnung | Beispiel Nr. | Peroraler $LD_{50}$-Wert in mg/kg | $ID_{30}$-Wert in mg/kg | Therapeutischer Index $\left(\frac{LD_{50}\text{-Wert}}{ID_{30}\text{-Wert}}\right)$ |
|---|---|---|---|---|
| Methyl-[6-(3,4-dichlorphenyl)-8-methyl-3,4-dihydro-2H,6H-pyrimido[2,1-b][1,3]thiazin-7-carboxylat] | 16 | 1 000 | 120 | 8,3 |
| Methyl-[8-methyl-6-(3,4,5-trimethoxyphenyl)-3,4-dihydro-2H,6H-pyrimido[2,1-b][1,3]thiazin-7-carboxylat] | 18 | > 1 000 | 110 | > 9,1 |
| Äthyl-[6-(4-dimethylamino-phenyl)-8-methyl-3,4-dihydro-2H,6H-pyrimido[2,1-b][1,3]thiazin-7-carboxylat] | 22 | 700 | 10 | 70 |

Fortsetzung der Tabelle III

| Untersuchte Verbindung Bezeichnung | Beispiel Nr. | Peroraler $LD_{50}$-Wert in mg/kg | $ID_{30}$-Wert in mg/kg | Therapeutischer Index $\left(\frac{LD_{50}\text{-Wert}}{ID_{30}\text{-Wert}}\right)$ |
|---|---|---|---|---|
| Indometacin | Vergleichs-substanz B | 22,5 | 3 | 7,5 |
| Penylbutazon | Vergleichs-substanz C | 1 000 | 40 | 25 |
| Acetylsalicylsäure | Vergleichs-substanz D | 1 350 | 200 | 6,8 |
| Paracetamol | Vergleichs-substanz E | 1 180 | 200 | 5,9 |

Aus der obigen Tabelle III geht hervor, daß die erfindungsgemäßen Verbindungen die in der Therapie angewandten Vergleichssubstanzen hinsichtlich der absoluten Dosis und/oder hinsichtlich des therapeutischen Indexes übertreffen.

Untersuchung der die Hexobarbital-Narkose potenzierenden Wirkung von 3,4-Di-(hydro)-2H,6H-pyrimido[2,1-b]-[1,3]thiazin-derivaten an Mäusen

Verfahrensweise

Die Untersuchungen wurden an aus jeweils 6 Mäusen bestehenden Gruppe durchgeführt. 1 Stunde nach der peroralen Behandlung wurden sowohl die Blindversuchsgruppe als auch die mit den erfindungsgemäßen Verbindungen behandelten Gruppen durch intravenöse Verabreichung von 40 mg/kg Hexobarbital zum Einschlafen gebracht.

Die Tiere, deren Schlafzeit die durchschnittliche Schlafzeit der Blindversuchsgruppe um das 2,5-fache überstieg, wurden als eine positive Reaktion zeigende Tiere angesehen. Mit den so transformierten Werten wurde der $ED_{50}$-Wert errechnet (modifizierte Verfahrensweise von Kaergaard Nielsen C. und Mitarbeitern, Arch. Int. Pharmacodyn, 2 [1967], Seiten 170ff).

Die Ergebnisse sind in der folgenden Tabelle IV zusammengestellt.

Tabelle IV

| Untersuchte Verbindung | | Peroraler $LD_{50}$-Wert in mg/kg | Die Hexobarbital-Narkose potenzierende Wirkung | |
|---|---|---|---|---|
| Bezeichnung | Beispiel Nr. | | Peroraler $ED_{50}$-Wert in mg/kg | Therapeutischer Index $\left(\frac{LD_{50}\text{-Wert}}{ED_{50}\text{-Wert}}\right)$ |
| Äthyl-[8-phenyl-6-(4-nitrophenyl)-3,4-dihydro-2H,6H-pyrimido[2,1-b][1,3]thiazin-7-carboxylat] | 13 | 1 000 | etwa 200 | etwa 5,0 |
| Methyl-[3,8-dimethyl-6-(3-nitrophenyl)-3,4-dihydro-2H,6H-pyrimido[2,1-b][1,3]thiazin-7-carboxylat] | 10 | >1 000 | 39 | > 25,6 |
| 6-(2-Fluor-6-chlorphenyl)-8-methyl-3,4-dihydro-2H,6H-pyrimido[2,1-b][1,3]thiazin-7-carbonsäureanilid | 6 | >1 000 | 25 | > 40,0 |

Fortsetzung der Tabelle IV

| Untersuchte Verbindung | | Peroraler $LD_{50}$-Wert in mg/kg | Die Hexobarbital-Narkose potenzierende Wirkung | |
|---|---|---|---|---|
| Bezeichnung | Beispiel Nr. | | Peroraler $ED_{50}$-Wert in mg/kg | Therapeutischer Index $\left(\frac{LD_{50}\text{-Wert}}{ED_{50}\text{-Wert}}\right)$ |
| Äthyl-6-(4-bromphenyl)-8-methyl-3,4-dihydro-2H,6H-pyrimido[2,1-b][1,3]thiazin-7-carboxylat | 11 | >1 000 | etwa 200 | >etwa 5,0 |
| Meprobamat | Vergleichssubstanz F | 1 100 | 260 | 4,2 |

Aus der obigen Tabelle IV geht hervor, daß die sedative Wirkung der erfindungsgemäßen Verbindungen die der Vergleichssubstanz sowohl hinsichtlich der absoluten Dosis als auch hinsichtlich des therapeutischen Indexes übertraf. Zur narkosepotenzierenden Wirkung kommt noch eine schwächere motilitätshemmende Wirkung hinzu.

Die erfindungsgemäßen Arzneimittel können in Form von den beziehungsweise die Wirkstoff(e) und 1 oder mehr inerte[n] nicht toxische[n], feste[n] und/oder flüssige[n] Träger- und/oder Hilfsstoff(e) enthaltenden Arzneimittelpräparaten vorliegen. Sie können zu Arzneimittelpräparaten zur peroralen Verabreichung, beispielsweise als Tabletten, überzogene Tabletten, Dragees, harte oder weiche Gelatinekapseln, Lösungen, Emulsionen oder Suspensionen, parenteralen Verabreichung, beispielsweise als Injektionslösungen, oder rektalen Verabreichung, beispielsweise als Suppositorien, zubereitet sein.

Die Herstellung der erfindungsgemäßen Arzneimittelpräparate kann nach bei der Arzneimittelherstellung an sich bekannten Verfahren durch Vermischen des beziehungsweise der Wirkstoffe[s] und des beziehungsweise der inerten anorganischen und/oder organischen, festen und/oder flüssigen Träger- und/oder Hilfsstoffe[s] und darauffolgendes Überführen des Gemisches in eine galenische Form durchgeführt werden.

Für Tabletten, überzogene Tabletten, Dragees und harte Gelatinekapseln kann beziehungsweise können als Trägerstoff(e) zum Beispiel Lactose, Maisstärke, Kartoffelstärke, Talk, Magnesiumcarbonat, Magnesiumstearat, Calciumcarbonat, Stearinsäure und/oder [ein] Salz(e) derselben dienen. Weiche Gelatinekapseln können als Trägerstoff(e) zum Beispiel 1 oder mehr Pflanzenöl(e), Fett(e), Wachs(e) und/oder Polyol(e) von entsprechender Konsistenz enthalten. Für Lösungen und Sirupe kann beziehungsweise können als Trägerstoff(e) zum Beispiel Wasser, 1 oder mehr Polyol(e), wie Polyäthylenglykol(e), Rohrzucker und/oder Glucose dienen. Injektionslösungen können als Trägerstoff(e) zum Beispiel Wasser, 1 oder mehr Alkohol(e), Polyol(e), Glycerin und/oder 1 oder mehr Pflanzenöl(e) enthalten. Für Suppositorien kann beziehungsweise können als Trägerstoff(e) zum Beispiel 1 oder mehr Öl(e), Wachs(e), Fett(e) und/oder Polyol(e) von entsprechender Konsistenz dienen.

Die erfindungsgemäßen Arzneimittelpräparate können ferner als Hilfsstoff(e) in der Arzneimittelherstellung übliche, zum Beispiel Netzmittel, Süßungs- und/oder Aromastoffe, Salze zur Veränderung des osmotischen Druckes und/oder Puffer, enthalten.

Die Verwendung der erfindungsgemäßen Verbindungen kann in der Weise erfolgen, daß der Patient mit einer wirksamen Dosis derselben behandelt wird. Die tägliche Dosis der erfindungsgemäßen Verbindungen kann innerhalb weiter Grenzen variieren und ist von mehreren Faktoren, zum Beispiel der Wirksamkeit des Wirkstoffes, dem Zustand und Alter des Patienten und dem Grad der Erkrankung, abhängig. Die perorale Dosis beträgt vorzugsweise 2 bis 500 mg/Tag. Es sei bemerkt, daß diese Dosen nur informativen Charakter haben und die zu verabreichende Dosis jeweils vom behandelten Arzt zu bestimmen ist.

Die erfindungsgemäßen Verbindungen sind in der Therapie vorzugsweise peroral, in Form von Tabletten oder Kapseln anzuwenden. Besonders vorteilhaft ist die Verwendung von 0,5 bis 100 mg Wirkstoff enthaltenden Tabletten oder Kapseln.

Die Erfindung wird an Hand der folgenden Beispiele näher erläutert.

**Beispiel 1**

Herstellung von Äthyl-(6-phenyl-8-methyl-3,4-dihydro-2H,6H-pyrimido[2,1-b][1,3]thiazin-7-carboxylat) sowie seines Hydrobromid- und Hydrochlorid-Salzes

27,6 g (0,1 Mol) Äthyl-(4-phenyl-6-methyl-1,2,3,4-tetrahydro-2-pyrimidinthion-5-carboxylat und 30,3 g (0,15 Mol) 1,3-Dibrompropan werden in Gegenwart von 27,6 g (0,2 Mol) Kaliumcarbonat und 2,0 g (0,012 Mol) Kaliumjodid in einem Gemisch aus 500 ml Methyläthylketon und 50 ml Dimethylformamid 15 Stunden lang unter Rückfluss zum Sieden erhitzt. Dann läßt man das Reaktionsgemisch auf Raumtemperatur abkühlen, es wird filtriert, und das Filtrat wird eingedampft. Der Rückstand wird aus Äthylacetat kristallisiert, abfiltriert und getrocknet. Man erhält 25,8 g (65 %) des Hydrobromidsalzes der Titelverbindung. Schmelzpunkt: 192 - 194 °C.

Basenbildung:

Das Hydrobromid-Salz wird in 530 ml Wasser aufgelöst, und durch Zugabe von Natriumhydrogencarbonat wird der pH der Lösung auf neutral eingestellt. Die abgeschiedenen Kristalle werden abfiltriert, mit Wasser gewaschen und getrocknet. Man erhält 19,5 g (95 %) der Base der Titelverbindung. Schmelzpunkt:

110 - 112 °C.

Bildung des salzsauren Salzes:

Die Base wird in 225 ml Äthylacetat gelöst, dann wird eine äquimolare Menge von Chlorwasserstoff enthaltendem Äthylalkohol [2,26 g (0,062 Mol) von Äthylalkohol absorbierter gasförmiger Chlorwasserstoff] zugetropft. Nach 1-stündigem Rühren wird die Suspension auf 5 °C gekühlt, filtriert, mit Äthylacetat gewaschen und getrocknet. Man erhält 21,3 g der Titelverbindung.
Bruttoformel: $C_{17}H_{20}N_2O_2S.HCl$, Molgewicht: 352,879.

| Elementaranalyse: | | | | | |
|---|---|---|---|---|---|
| berechnet: | C = 57,68 %; | H = 6,0 %; | N = 7,94 %; | S = 9,08 %; | $Cl^-$ = 10,05 %; |
| gemessen: | C = 58,87 %; | H = 6,17 %; | N = 7,87 %; | S = 9,18 %; | $Cl^-$ = 10,25 %. |

## Beispiel 2

Herstellung von Äthyl-[6-(2-fluor-6-chlorphenyl)-8-methyl-3,4-dihydro-2H,6H-pyrimido[2,1-b][1,3]thiazin-7-carboxylat]

32,9 (0,1 Mol) Äthyl-[4-(2-fluor-6-chlorphenyl)-6-methyl-1,2,3,4-tetrahydro-2-pyrimidinthion-5-carboxylat] und 30,3 g (0,15 Mol) 1,3-Dibromopropan werden unter den im Beispiel 1 beschriebenen Bedingungen 20 Stunden lang umgesetzt. Der Rückstand wird aus Wasser kristallisiert, abfiltriert und getrocknet. Man erhält 35 g (94,9 %) der Ti telverbindung.
Schmelzpunkt: 129 - 131 °C.
Bruttoformel: $C_{17}H_{18}ClFN_2O_2S$, Molgewicht : 368,855.

| Elementaranalyse: | | | | | |
|---|---|---|---|---|---|
| berechnet: | C = 55,36 %; | H = 4,92 %; | N = 7,59 %; | S = 8,69 %; | Cl = 9,61 %; |
| gemessen: | C = 55,82 %; | H = 5,00 %; | N = 7,57 %; | S = 8,76 %; | Cl = 9,57 %. |

## Beispiel 3

Herstellung von Äthyl-[6-(3,4-dichlorphenyl)-8-methyl-3,4-dihydro-2H,6H-pyrimido[2,1-b][1,3]thiazin-7-carboxylat]

34,5 g (0,1 Mol) Äthyl-[4-(3,4-dichlorphenyl)-6-methyl-1,2,3,4-tetrahydro-2-pyrimidinthion-5-carboxylat] und 30,3 g (0,15 Mol) 1,3-Dibrompropan werden unter den im Beispiel 1 beschriebenen Bedingungen 26 Stunden lang umgesetzt. Der Rückstand wird aus Wasser kristallisiert, abfiltriert und getrocknet. Man erhält 37 g (97,0 %) der Titelverbindung.
Schmelzpunkt: 116 - 118 °C.
Bruttoformel: $C_{17}H_{18}NCl_2N_2O_2S$, Molgewicht: 385,315.

| Elementaranalyse: | | | | | |
|---|---|---|---|---|---|
| errechnet:<br>gemessen: | C=52,99 %;<br>C=52,71 %; | H=4,71 %;<br>H=4,71 %; | N=7,27 %;<br>N=7,27 %; | S=8,32 %;<br>S=8,32 %; | Cl=18,40 %;<br>Cl=18,40 %. |

**Beispiel 4**

Herstellung von Methyl-[6-(3-nitrophenyl)-8-methyl-3,4-dihydro-2H,6H-pyrimido-[2,1-b][1,3]thiazin-7-carboxylat]

30,7 g (0,1 Mol) Methyl-[4-(3-nitrophenyl)-6-methyl-1,2,3,4-tetrahydro-2-pyrimidinthion-5-carboxylat] und 30,3 g (0,15 Mol) 1,3-Dibrompropan werden unter den in Beispiel 1 beschriebenen Bedingungen 13 Stunden lang umgesetzt. Der Rückstand wird aus Wasser kristallisiert, abfiltriert und getrocknet. Man erhält 29,2 g (84 %) der Titelverbindung.
Schmelzpunkt: 178 - 180 °C.
Bruttoformel: $C_{16}H_{17}N_3O_4S$, Molgewicht: 347,391.

| Elementaranalyse: | | | | |
|---|---|---|---|---|
| berechnet:<br>gemessen: | C=55,32 %;<br>C=55,27 %; | H=4,93 %;<br>H=4,86 %; | N=12,09 %;<br>N=12,03 %; | S=9,23 %;<br>S=9,11 %. |

**Beispiel 5**

Herstellung von 6-Phenyl-8-methyl-3,4-dihydro-2H,6H-pyrimido-[2,1-b][1,3]thiazin-7-carbonsäureanilid und seines Hydrochlorids

32,3 g (0,1 Mol) 4-Phenyl-6-methyl-1,2,3,4-tetrahydro-2-pyrimidinthion-5-carbonsäureanilid und 30,3 g (0,15 mol) 1,3-Dibrompropan werden unter den im Beispiel 1 beschriebenen Bedingungen 6 Stunden lang umgesetzt. Der Rückstand wird aus Äthylalkohol kristallisiert, abfiltriert und getrocknet. Man erhält 21,8 g (60 %) der Base der Titelverbindung.
Schmelzpunkt: 222 - 224 °C.

Bildung des salzsauren Salzes:

18,2 g (0,05 Mol) der Base werden in 170 ml Äthylacetat suspendiert, dann wird eine äquimolare Menge Chlorwasserstoff enthaltender Äthylalkohol zugetropft. Nach 1-stündigem Rühren wird die Suspension auf 5 °C abgekühlt, filtriert, mit Äthylacetat gewaschen und getrocknet. Man erhält 17,6 g (88 %) der Titelverbindung.
Schmelzpunkt: 206 - 209 °C.
Bruttoformel: $C_{21}H_{21}N_3O_5.HCl$, Molgewicht: 399,938.

| Elementaranalyse: | | | | | |
|---|---|---|---|---|---|
| berechnet:<br>gemessen: | C=63,07 %;<br>C=62,59 %; | H=5,54 %;<br>H=5,69 %; | N=10,51 %;<br>N=10,20 %; | S=8,02 %;<br>S=7,84 %; | $Cl^-$=8,86 %;<br>$Cl^-$=8,75 %. |

**Beispiel 6**

Herstellung von 6-(2-Fluor-6-chlorphenyl)-8-methyl-3,4-dihydro-2H,6H-pyrimido[2,1-b][1,3]thiazin-7-carbonsäureanilid

37,6 g (0,1 Mol) 4-(2-Fluor-6-chlorphenyl)-6-methyl-1,2,3,4-tetrahydro-2-pyrimidinthion-5-carbonsäureanilid und 23,2 g (0,115 Mol) 1,3-Dibrompropan werden in Gegenwart von 13,8 g (0,1 Mol) Kaliumcarbonat in 200 ml Dimethylformamid 7 Stunden lang bei 70 °C umgesetzt. Dann lässt man das Reaktionsgemisch auf Raumtemperatur abkühlen, es wird filtriert, und das Filtrat wird unter Vakuum eingedampft. Der Rückstand wird aus Wasser kristallisiert, abfiltriert und getrocknet. Man erhält 40,3 g (97 %) der Titelverbindung.
Schmelzpunkt: 210 - 215 °C.
Bruttoformel: $C_{21}H_{19}ClFN_3OS$, Molgewicht: 415,914.

| Elementaranalyse: | | | | | |
|---|---|---|---|---|---|
| berechnet:<br>gemessen: | C=60,65 %;<br>C=59,68 %; | H=4,60 %;<br>H=4,59 %; | N=10,10 %;<br>N= 9,94 %; | S=7,71 %;<br>S=7,71 %; | Cl=8,52 %;<br>Cl=8,49 %. |

**Beispiel 7**

Herstellung von Äthyl-[8-methyl-6-(4-methoxy-phenyl)-3,4-dihydro-2H,6H-pyrimido[2,1-b][1,3]thiazin-7-carboxylat]

30,6 g (0,1 Mol) Äthyl-[6-methyl-4-(4-methoxy-phenyl)-1,2,3,4-tetrahydro-2-pyrimidinthion-5-carboxylat] und 30,3 g (0,15 Mol) 1,3-Dibrompropan werden unter den im Beispiel 1 beschriebenen Bedingungen 13 Stunden lang umgesetzt. Der Rückstand wird aus Isopropylalkohol kristallisiert, abfiltriert und getrocknet. Man erhält 18 g (52 %) der Titelverbindung.
Schmelzpunkt: 148 - 150 °C.
Bruttoformel: $C_{18}H_{22}N_2O_3S$, Molgewicht: 346,447.

| Elementaranalyse: | | | | |
|---|---|---|---|---|
| berechnet:<br>gemessen: | C=62,40 %;<br>C=62,33 %; | H=6,40 %;<br>H=6,33 %; | N=8,09 %;<br>N=8,13 %; | S=9,25 %;<br>S=9,10 %. |

**Beispiel 8**

Herstellung von Methyl-[6-(4-chlor-3-nitrophenyl)-8-methyl-3,4-dihydro-2H,6H-pyrimido[2,1-b][1,3]thiazin-7-carboxylat]

34,2 g (0,1 Mol) Methyl-[4-(4-chlor-3-nitrophe nyl)-6-methyl-1,2,3,4-tetrahydro-2-pyrimidinthion-5-carboxylat] und 30,3 g (0,15 Mol) 1,3-Dibrompropan werden unter den im Beispiel 1 beschriebenen Bedingungen 13 Stunden lang umgesetzt. Der Rückstand wird aus Wasser kristallisiert abfiltriert und getrocknet. Man erhält 37,4 g (98 %) der Titelverbindung.
Schmelzpunkt: 146 - 148 °C.

Bruttoformel: $C_{16}H_{16}ClN_3O_4S$, Molgewicht: 381,834.

| Elementaranalyse: | | | | | |
|---|---|---|---|---|---|
| berechnet:<br>gemessen: | C=50,33 %;<br>C=49,00 %; | H=4,22 %;<br>H=4,33 %; | N=11,0 %;<br>N=10,78 %; | Cl=9,28 %;<br>Cl=9,32 %; | S=8,40 %;<br>S=8,07 %. |

**Beispiel 9**

Herstellung von Methyl-[3,8-dimethyl-6-(4-chlor-3-nitrophenyl)-3,4-dihydro-2H,6H-pyrimido[2,1-b][1,3]thiazin-7-carboxylat]

34,2 g (0,1 Mol) Methyl-[4-(4-chlor-3-nitrophenyl)-6-methyl-1,2,3,4-tetrahydro-2-pyrimidinthion-5-carboxylat] und 19,7 g (0,115 Mol) 2-Methyl-1,3-chlor-brom-propan werden unter den im Beispiel 6 beschriebenen Bedingungen 16 Stunden lang umgesetzt. Der Rückstand wird aus Wasser kristallisiert, abfiltriert und getrocknet. Man erhält 29,7 g (75 %) der Titelverbindung.
Schmelzpunkt: 139 - 142 °C.
Bruttoformel: $C_{17}H_{18}ClN_3O_4S$, Molgewicht: 395,861.

| Elementaranalyse: | | | | | |
|---|---|---|---|---|---|
| berechnet:<br>gemessen: | C=51,58 %;<br>C=51,12 %; | H=4,58 %;<br>H=4,46 %; | N=10,61 %;<br>N=10,61 %; | S=8,10 %;<br>S=8,06 %. | Cl=8,96 %;<br>Cl=8,97 %. |

**Beispiel 10**

Herstellung von Methyl-[3,8-dimethyl-6-(3-nitrophenyl)-3,4-dihydro-2H,6H-pyrimido[2,1-b][1,3]thiazin-7-carboxylat]

30,7 g (0,1 Mol) Methyl-[4-(3-nitrophenyl)-6-methyl-1,2,3,4-tetrahydro-2-pyrimidinthion-5-carboxylat] und 19,7 g (0,115 Mol) 2-Methyl-1,3-chlor-brompropan werden unter den im Beispiel 6 beschriebenen Bedingungen 24 Stunden lang bei 100 °C umgesetzt. Der Rückstand wird aus Wasser kristallisiert, abfiltriert und getrocknet. Man erhält 31,4 g (87,1 %) der Titelverbindung.
Schmelzpunkt: 145 - 150 °C.
Bruttoformel: $C_{17}H_{18}N_3O_4S$, Molgewicht: 360,411.

| Elementaranalyse: | | | | |
|---|---|---|---|---|
| berechnet:<br>gemessen: | C=56,65 %;<br>C=56,78 %; | H=5,03 %;<br>H=5,34 %; | N=11,66 %;<br>N=11,54 %; | S=8,89 %;<br>S=9,06 %. |

**Beispiel 11**

Herstellung von Äthyl-6-(4-bromphenyl)-8-methyl-3,4-dihydro-2H,6H-pyrimido[2,1-b][1,3]thiazin-7-carboxylat

35,5 g (0,1 Mol) Äthyl-[4-(4-bromphenyl)-6-methyl-1,2,3,4-tetrahydro-2-pyrimidinthion-5-carboxylat] und 30,3 g (0,15 Mol) 1,3-Dibrompropan werden unter den im Beispiel 1 beschriebenen Bedingungen 13 Stunden lang umgesetzt. Der Rückstand wird aus Wasser kristallisiert, abfiltriert und getrocknet. Man erhält 28,2 g (71,3 %) der Titelverbindung.
Schmelzpunkt: 150 - 153 °C.
Bruttoformel: $C_{17}H_{19}BrN_2O_2S$, Molgewicht: 395,393.

| Elementaranalyse: | | | | | |
|---|---|---|---|---|---|
| berechnet:<br>gemessen: | C=51,65 %;<br>C=52,00 %; | H=4,84 %;<br>H=4,93 %; | N=7,09 %;<br>N=7,42 %; | Br=20,21 %;<br>Br=20,06 %; | S=8,11 %;<br>S=7,94 %. |

**Beispiel 12**

Herstellung von Äthyl-[8-phenyl-6-(2-fluor-6-chlorphenyl)-3,4-dihydro-2H,6H-pyrimido[2,1-b][1,3]thiazin-7-carboxylat]

39,1 g (0,1 Mol) Äthyl-[6-phenyl-4-(2-fluor-6-chlorphenyl)-1,2,3,4-tetrahydro-2-pyrimidinthion-5-carboxylat] und 23,2 g (0,115 Mol) 1,3-Dibrompropan werden unter den im Beispiel 6 beschriebenen Bedingungen 5 Stunden lang umgesetzt. Der Rückstand wird aus Wasser kristalli siert, abfiltriert und getrocknet. Man erhält 39,6 g (92 %) der Titelverbindung.
Schmelzpunkt: 168 - 170 °C.
Bruttoformel: $C_{22}H_{20}ClFN_2O_2S$, Molgewicht: 430,926.

| Elementaranalyse: | | | | | |
|---|---|---|---|---|---|
| berechnet:<br>gemessen: | C=61,32 %;<br>C=60,40 %; | H=4,68 %;<br>H=4,64 %; | N=6,50 %;<br>N=6,49 %; | S=7,44 %;<br>S=7,44 %; | Cl=8,23 %;<br>Cl=8,12 %. |

**Beispiel 13**

Herstellung von Äthyl-[8-phenyl-6-(4-nitrophenyl)-3,4-dihydro-2H,6H-pyrimido[2,1-b][1,3]thiazin-7-carboxylat]

38,3 g (0,1 Mol) Äthyl-[6-phenyl-4-(4-nitrophenyl)-1,2,3,4-tetrahydro-2-pyrimidinthion-5-carboxylat] und 30,3 g (0,15 Mol) 1,3-Dibrompropan werden unter den im Beispiel 1 beschriebenen Bedingungen 6 Stunden lang umgesetzt. Der Rückstand wird aus Wasser kristallisiert, abfiltriert und getrocknet. Man erhält 19,1 g (45 %) der Titelverbindung.
Schmelzpunkt: 190 - 192 °C.
Bruttoformel: $C_{22}H_{21}N_3O_4S$, Molgewicht: 423,49.

| Elementaranalyse: | | | | |
|---|---|---|---|---|
| berechnet:<br>gemessen: | C=62,40 %;<br>C=62,77 %; | H=5,0 %;<br>H=5,6 %; | N=9,92 %;<br>N=9,73 %; | S=7,54 %;<br>S=7,47 %. |

**Beispiel 14**

Herstellung von Methyl-(6,8-dimethyl-3,4-dihydro-2H,6H-pyrimido[2,1-b][1,3]-thiazin-7-carboxylat)

20,0 g (0,1 Mol) Methyl-(4,6-dimethyl-1,2,3,4-tetrahydro-2-pyrimidinthion-5-carboxylat) und 30,3 g (0,15 Mol) 1,3-Dibrompropan werden in Gegenwart von 27,6 g (0,2 Mol) Kaliumcarbonat und 2,0 g (0,012 Mol) Kaliumjodid in einem Gemisch aus 500 ml Aceton und 50 ml Dimethylformamid 32 Stunden lang bei Siedetemperatur umgesetzt. Dann lässt man das Reaktionsgemisch auf Raumtemperatur abkühlen, danach wird filtriert, und das Filtrat wird ein gedampft. Der Rückstand wird aus Wasser kristallisiert, abfiltriert und getrocknet. Man erhält 19,2 g (80 %) der Titelverbindung.
Schmelzpunkt: 92 - 94 °C.
Bruttoformel: $C_{11}H_{16}N_2O_2S$, Molgewicht: 240,323.

| Elementaranalyse: | | | | |
|---|---|---|---|---|
| berechnet:<br>gemessen: | C = 54,98 %;<br>C = 55,37 %; | H = 6,71 %;<br>H = 6,78 %; | N = 11,66 %;<br>N = 11,35 %; | S = 13,34 %;<br>S = 13,22 %. |

**Beispiel 15**

Herstellung von Methyl-[8-methyl-6-(4-methoxy-phenyl)-3,4-dihydro-2H,6H-pyrimido[2,1-b][1,3]thiazin-7-carboxylat]

29,2 g (0,1 Mol) Methyl-[6-methyl-4-(4-methoxy-phenyl)-1,2,3,4-tetrahydro-2-pyrimidinthion-5-carboxylat] und 30,3 g (0,15 Mol) 1,3-Dibrompropan werden unter den im Beispiel 14 beschriebenen Bedingungen 36 Stunden umgesetzt. Der Rückstand wird aus Isopropylalkohol kristallisiert, abfiltriert und getrocknet. Man erhält 27,9 g (83,9 %) der Titelverbindung.
Schmelzpunkt: 185 - 186 °C.
Bruttoformel: $C_{17}H_{20}N_2O_3S$, Molgewicht: 332,42.

| Elementaranalyse: | | | | |
|---|---|---|---|---|
| berechnet:<br>gemessen: | C = 61,42 %;<br>C = 61,95 %; | H = 6,06 %;<br>H = 6,30 %; | N = 8,43 %;<br>N = 8,40 %; | S = 9,64 %;<br>S = 9,46 %. |

**Beispiel 16**

Herstellung von Methyl-[6-(3,4-dichlorphenyl)-8-methyl-3,4-dihydro-2H,6H-pyrimido[2,1-b][1,3]thiazin-7-carboxylat]

33,1 g (0,1 Mol) Methyl-[4-(3,4-dichlorphenyl)-6-methyl-1,2,3,4-tetrahydro-2-pyrimidinthion-5-carboxylat] und 30,3 g (0,15 Mol) 1,3-Dibrompropan werden unter den im Beispiel 14 beschriebenen Bedingungen 23 Stunden umgesetzt. Der Rückstand wird aus Wasser kristallisiert, abfiltriert und getrocknet. Man erhält 35,3 g (95 %) der Titelverbindung.
Schmelzpunkt: 151 - 152 °C.
Bruttoformel $C_{16}H_{16}Cl_2N_2O_2S$, Molgewicht: 371,288.

| Elementaranalyse: | | | | | |
|---|---|---|---|---|---|
| berechnet:<br>gemessen: | C=51,76 %;<br>C=52,02 %; | H=4,34 %;<br>H=4,43 %; | N=7,54 %;<br>N=7,62 %; | S=8,63 %;<br>S=8,71 %; | Cl=19,10 %;<br>Cl=18,38 %. |

**Beispiel 17**

Herstellung von Äthyl-[8-methyl-6-(3-nitrophenyl)-3,4-dihydro-2H,6H-pyrimido[2,1-b][1,3]thiazin-7-carboxylat]

32,1 g (0,1 Mol) Äthyl-[6-methyl-4-(3-nitrophenyl)-1,2,3,4-tetrahydro-2-pyrimidinthion-5-carboxylat] und 30,3 g (0,15 Mol) 1,3-Dibrompropan werden unter den im Beispiel 14 beschriebenen Bedingungen 30 Stunden lang umgesetzt. Der Rückstand wird aus Wasser kristallisiert, abfiltriert und getrocknet. Man erhält 33,6 g (93 %) der Titelverbindung.
Schmelzpunkt: 163 - 165 °C.
Bruttoformel: $C_{17}H_{19}N_3O_4S$, Molgewicht: 361,419.

| Elementaranalyse: | | | | |
|---|---|---|---|---|
| berechnet:<br>gemessen: | C=56,49 %;<br>C=56,89 %; | H=5,30 %;<br>H=5,05 %; | N=11,63 %;<br>N=11,48 %; | S=8,87 %;<br>S=8,76 %. |

**Beispiel 18**

Herstellung von Methyl-[8-methyl-6-(3,4,5-trimethoxyphenyl)-3,4-dihydro-2H,6H-pyrimido[2,1-b][1,3]thiazin-7-carboxylat]

35,2 g (0,1 Mol) Methyl-[6-methyl-4-(3,4,5-trimethoxyphenyl)-1,2,3,4-tetrahydro-2-pyrimidinthion-5-carboxylat] und 30,3 g (0,15 Mol) 1,3-Dibrompropan werden unter den im Beispiel 14 beschriebenen Bedingungen 32 Stunden lang umgesetzt. Der Rückstand wird aus Isopropylalkohol kristallisiert, abfiltriert und getrocknet. Man erhält 19,6 g (50 %) der Titelverbindung.
Schmelzpunkt: 137 - 138 °C.
Bruttoformel: $C_{19}H_{24}N_2O_5S$, Molgewicht: 392,473.

| Elementaranalyse: | | | | |
|---|---|---|---|---|
| berechnet:<br>gemessen: | C=58,15 %;<br>C=57,28 %; | H=6,16 %;<br>H=5,96 %; | N=7,14 %;<br>N=7,02 %; | S=8,17 %;<br>S=8,01 %. |

**Beispiel 19**

Herstellung von Äthyl-[8-phenyl-6-(4-methoxyphenyl)-3,4-dihydro-2H,6H-pyrimido[2,1-b][1,3]thiazin-7-carboxylat]

36,8 g (0,1 Mol) Äthyl-[6-phenyl-4-(4-methoxy)phenyl)-1,2,3,4-tetrahydro-2-pyrimidinthion-5-carboxylat]

und 30,3 g (0,15 mol) 1,3-Dibrompropan werden unter den im Beispiel 1 beschriebenen Bedingungen 9 Stunden lang umgesetzt. Die als Rückstand verbliebenen Kristalle werden abfiltriert, mit Isopropylalkohol gewaschen und getrocknet. Man erhält 32,7 g (80%) der Titelverbindung.
Schmelzpunkt: 180 - 182 °C.
Bruttoformel: $C_{23}H_{24}N_2O_3S$, Molgewicht: 408,518.

| Elementaranalyse: | | | | |
|---|---|---|---|---|
| berechnet:<br>gemessen: | C=67,62 %;<br>C=67,33 %; | H=5,92 %;<br>H=5,90 %; | N=6,86 %;<br>N=6,93 %; | S=7,85 %;<br>S=7,69 %. |

**Beispiel 20**

Herstellung von Methyl-[8-methyl-6-(4-methylphenyl)-3,4-dihydro-2H,6H-pyrimido[2,1-b][1,3]thiazin-7-carboxylat]

27,6 g (0,1 Mol) Methyl-[6-methyl-4-(4-methylphenyl)-1,2,3,4-tetrahydro-2-pyrimidinthion-5-carboxylat] und 30,3 g (0,15 Mol) 1,3-Dibrompropan werden unter den im Beispiel 14 beschriebenen Bedingungen 25 Stunden lang umgesetzt. Der Rückstand wird mit wenig Aceton gewaschen, abfiltriert und getrocknet. Man erhält 24,7 g (78 %) der Titelverbindung.
Schmelzpunkt: 184 - 186 °C.
Bruttoformel: $C_{17}H_{20}N_2O_2S$, Molgewicht: 316,404.

| Elementaranalyse: | | | | |
|---|---|---|---|---|
| berechnet:<br>gemessen: | C=64,53 %;<br>C=64,67 %; | H=6,37 %;<br>H=6,46 %; | N=8,85 %;<br>N=8,89 %; | S=10,13 %;<br>S=10,11 %. |

**Beispiel 21**

Herstellung von Äthyl-[8-methyl-6-(3,4,5-trimethoxyphenyl)-3,4-dihydro-2H,6H-pyrimido[2,1-b][1,3]thiazin-7-carboxylat]

36,6 g (0,1 Mol) Äthyl-[6-methyl-4-(3,4,5-trimethoxyphenyl)-1,2,3,4-tetrahydro-2-pyrimidinthion-5-carboxylat] und 30,3 g (0,15 Mol) 1,3-Dibrompropan werden unter den im Beispiel 14 beschriebenen Bedingungen 28 Stunden lang umgesetzt. Der Rückstand wird aus Wasser kristallisiert, abfiltriert und getrocknet. Man erhält 34 g (83,6 %) der Titelverbindung.
Schmelzpunkt: 104 - 105 °C.
Bruttoformel: $C_{20}H_{26}N_2O_5S$, Molgewicht: 404,480.

| Elementaranalyse: | | | | |
|---|---|---|---|---|
| berechnet:<br>gemessen: | C=59,09 %;<br>C=58,94 %; | H=6,45 %;<br>H=6,50 %; | N=6,89 %;<br>N=6,82 %; | S=7,88 %;<br>S=7,76 %. |

**Beispiel 22**

Herstellung von Äthyl-[6-(4-dimethylamino-phenyl)-8-methyl-3,4-dihydro-2H,6H-pyrimido[2,1-b][1,3]thiazin-7-carboxylat]

31,9 g (0,1 Mol) Äthyl-[4-(dimethylamino-phenyl)-6-methyl-1,2,3,4-tetrahydro-2-pyrimidinthion-5-carboxylat] und 30,3 g (0,15 Mol) 1,3-Dibrompropan werden unter den im Beispiel 14 beschriebenen Bedingungen 30 Stunden lang umgesetzt. Der Rückstand wird aus Wasser kristallisiert, abfiltriert und getrocknet. Man erhält 31,3 g (87,1 %) der Titelverbindung.
Schmelzpunkt: 128 - 130 °C.
Bruttoformel: $C_{19}H_{24}N_3O_2S$, Molgewicht: 358,335.

| Elementaranalyse: | | | | |
|---|---|---|---|---|
| berechnet:<br>gemessen: | C=63,48 %;<br>C=63,91 %; | H=7,01 %;<br>H=6,97 %; | N=11,69 %;<br>N=11,69 %; | S=8,92 %;<br>S=8,80 %. |

## Beispiel 23

Herstellung von Äthyl-[6-äthyl-8-phenyl-3,4-dihydro-2H,6H-pyrimido[2,1-b][1,3]thiazin-7-carboxylat]

29 g (0,1 Mol) Äthyl-(4-äthyl-6-phenyl-1,2,3,4-tetrahydro-2-pyrimidinthion-5-carboxylat) und 30,3 g (0,15 Mol) 1,3-Dibrompropan werden unter den im Beispiel 14 beschriebenen Bedingungen 28 Stunden lang umgesetzt. Der Rückstand wird aus Wasser kristallisiert, abfiltriert und getrocknet. Man erhält 27 g (81,7 %) der Titelverbindung.
Schmelzpunkt: 74 - 76 °C.
Bruttoformel: $C_{18}H_{22}N_2O_2S$, Molgewicht: 330,448.

| Elementaranalyse: | | | | |
|---|---|---|---|---|
| berechnet:<br>gemessen: | C=65,43 %;<br>C=64,11 %; | H=6,71 %;<br>H=6,61 %; | N=8,48 %;<br>N=8,33 %; | S=9,70 %;<br>S=9,41 %. |

## Beispiel 24

Herstellung von Methyl-[6-phenyl-8-methyl-3,4-dihydro-2H,6H-pyrimido[2,1-b][1,3]thiazin-7-carboxylat]

26,2 g (0,1 Mol) Methyl-(4-phenyl-6-methyl-1,2,3,4-tetrahydro-2-pyrimidinthion-5-carboxylat) und 30,3 g (0,15 Mol) 1,3-Dibrompropan werden unter den im Beispiel 14 beschriebenen Bedingungen 17 Stunden lang umgesetzt. Der Rückstand wird aus Wasser kristallisiert, abfiltriert und getrocknet. Man erhält 19 g (62,8 %) der Titelverbindung.
Schmelzpunkt: 194 - 196 °C.
Bruttoformel: $C_{16}H_{18}N_2O_2S$, Molgewicht: 302,393.

| Elementaranalyse: | | | | |
|---|---|---|---|---|
| berechnet:<br>gemessen: | C=63,55 %;<br>C=62,98 %; | H=6,00 %;<br>H=5,91 %; | N=9,26 %;<br>N=9,02 %; | S=10,60 %;<br>S=10,25 %. |

**Beispiel 25**

Herstellung von Methyl-[8-methyl-6-(2-methoxy-phenyl)-3,4-dihydro-2H,6H-pyrimido[2,1-b][1,3]thiazin-7-carboxylat]

29,3 g (0,1 Mol) Methyl-[6-methyl-4-(2-methoxy phenyl)-1,2,3,4-tetrahydro-2-pyrimidinthion-5-carboxylat] und 30,3 g (0,15 Mol) 1,3-Dibrompropan werden unter den im Beispiel 1 beschriebenen Bedingungen 15 Stunden lang umgesetzt. Nach dem Eindampfen werden die abgeschiedenen Kristalle abfiltriert, mit wenig Äther gewaschen und getrocknet. Man erhält 20,9 g (62,9 %) der Titelverbindung.
Schmelzpunkt: 133 - 135 °C.
Bruttoformel: $C_{17}H_{20}N_2O_3S$, Molgewicht: 332,42.

| Elementaranalyse: | | | | |
|---|---|---|---|---|
| berechnet: | C=61,42 %; | H=6,06 %; | N=8,43 %; | S=9,64 %; |
| gemessen: | C=62,12 %; | H=6,20 %; | N=8,34 %; | S=9,48 %. |

**Beispiel 26**

Herstellung von Äthyl-[3,8-dimethyl-6-(3-nitrophenyl)-3,4-dihydro-2H,6H-pyrimido[2,1-b][1,3]thiazin-7-carboxylat]

32,1 g (0,1 Mol) Äthyl-[6-methyl-4-(3-nitrophenyl)-1,2,3,4-tetrahydro-2-pyrimidinthion-5-carboxylat] und 19,7 g (0,115 Mol) 2-Methyl-1,3-chlor-brompropan werden unter den im Beispiel 6 beschriebenen Bedingungen 15 Stunden lang umgesetzt. Der Rückstand wird aus Wasser kristallisiert, abfiltriert und getrocknet. Man erhält 19,5 g (51,9 %) der Titelverbindung.
Schmelzpunkt: 124 - 126 °C.
Bruttoformel: $C_{18}H_{21}N_3O_4S$, Molgewicht: 375,446.

| Elementaranalyse: | | | | |
|---|---|---|---|---|
| berechnet: | C=57,58 %; | H=5,64 %; | N=11,19 %; | S=8,54 %; |
| gemessen: | C=56,48 %; | H=5,73 %; | N=11,18 %; | S=8,73 %. |

**Beispiel 27**

Herstellung von Methyl-[6-(4-dimethylamino-phenyl)-8-methyl-3,4-dihydro-2H,6H-pyrimido[2,1-b][1,3]thiazin-7-carboxylat]

30,5 g (0,1 Mol) Methyl-[4-(4-dimethylamino-phenyl)-6-methyl-1,2,3,4-tetrahydro-2-pyrimidinthion-5-carboxylat] und 30,3 g (0,15 Mol) 1,3-Dibrompropan werden un ter den im Beispiel 14 beschriebenen Bedingungen 32 Stunden lang umgesetzt. Der Rückstand wird aus Wasser kristallisiert, abfiltriert und getrocknet. Man erhält 26,6 g (77 %) der Titelverbindung.
Schmelzpunkt: 128 - 130 °C.
Bruttoformel: $C_{18}H_{23}N_3O_2S$, Molgewicht: 345,463.

| Elementaranalyse: | | | | |
|---|---|---|---|---|
| berechnet:<br>gemessen: | C=62,58 %;<br>C=62,50 %; | H=6,71 %;<br>H=6,76 %; | N=12,16 %;<br>N=12,11 %; | S=9,28 %;<br>S=9,14 %. |

**Beispiel 28**

Herstellung von Äthyl-[6-(4-chlor-3-nitrophenyl)-8-methyl-3,4-dihydro-2H,6H-pyrimido[2,1-b][1,3]thiazin-7-carboxylat]

35,6 g (0,1 Mol) Äthyl-[4-(4-chlor-3-nitrophenyl)-6-methyl-1,2,3,4-tetrahydro-2-pyrimidinthion-5-carboxylat] und 30,3 g (0,15 Mol) 1,3-Dibrompropan werden unter den im Beispiel 14 beschriebenen Bedingungen 13 Stunden lang umgesetzt. Der Rückstand wird aus Wasser kristallisiert, abfiltriert und getrocknet. Man erhält 36,4 g (92 %) der Titelverbindung.
Schmelzpunkt: 126 - 128 °C.
Bruttoformel: $C_{17}H_{18}ClN_3O_4S$, Molgewicht: 395,861.

| Elementaranalyse: | | | | | |
|---|---|---|---|---|---|
| berechnet:<br>gemessen: | C=51,58 %;<br>C=51,08 %; | H=4,58 %;<br>H=4,65 %; | N=10,61 %;<br>N=10,32 %; | Cl=8,96 %;<br>Cl=8,95 %; | S=8,10 %;<br>S=8,00 %. |

**Beispiel 29**

Herstellung von Methyl-[6-(3-hydroxy-4-methoxyphenyl)-8-methyl-3,4-dihydro-2H,6H-pyrimido[2,1-b][1,3]-thiazin-7-carboxylat]

30,8 g (0,1 Mol) Methyl-[4-(3-hydroxy-4-methoxy-phenyl)-6-methyl-1,2,3,4-tetrahydro-2-pyrimidinthion-5-carboxylat] und 30,3 g (0,15 Mol) 1,3-Dibrompropan werden unter den im Beispiel 14 beschriebenen Bedingungen 30 Stunden lang umgesetzt. Danach wird gekühlt, filtriert, mit Wasser gewaschen und getrocknet. Man erhält 21,9 g (65,1 %) der Titelverbindung.
Schmelzpunkt: 275 - 277 °C.
Bruttoformel: $C_{16}H_{20}N_2O_4S$, Molgewicht: 336,408.

| Elementaranalyse: | | | | |
|---|---|---|---|---|
| berechnet:<br>gemessen: | C=57,13 %;<br>C=56,98 %; | H=5,99 %;<br>H=6,05 %; | N=8,33 %;<br>N=8,28 %; | S=9,53 %;<br>S=9,45 %. |

**Beispiel 30**

Herstellung von Methyl-(4,6-dimethyl-1,2,3,4-tetrahydro-2-pyrimidinthion-5-carboxylat)

13,2 g (0,3 Mol) Acetaldehyd, 22,8 g (0,3 Mol) Thioharnstoff und 34,8 g (0,3 Mol) Acetessigsäuremethylester werden in 100 ml 15 Gew.-% Chlorwasserstoff enthaltendem Isopropylalkohol (15 g von 100 ml

Isopropylalkohol absorbierter gasförmiger Chlorwasserstoff) bei Raumtemperatur 12 Stunden lang umgesetzt.
Nach Ablauf der Reaktion wird die Suspension gekühlt, filtriert, mit Isopropylalkohol gewaschen und getrocknet. Man erhält 30 g (50 %) der Titelverbindung.
Schmelzpunkt: 203 - 206 °C.
Bruttoformel: $C_8H_{12}N_2O_2S$, Molgewicht: 200,257.

| Elementaranalyse: | | | | |
|---|---|---|---|---|
| berechnet:<br>gemessen: | C=47,98 %;<br>C=48,05 %; | H=6,04 %;<br>H=5,98 %; | N=13,99 %;<br>N=13,87 %; | S=16,0 %;<br>S=15,59 %. |

**Beispiel 31**

Herstellung von Methyl-(4-äthyl-6-methyl-1,2,3,4-tetrahydro-2-pyrimidinthion-5-carboxylat)

17,4 g (0,3 Mol) Propionaldehyd, 22,8 g (0,3 Mol) Thioharnstoff und 34,8 g (0,3 Mol) Acetessigsäuremethylester werden unter den im Beispiel 30 angegebenen Bedingungen 35 Stunden lang umgesetzt. Nach Kühlen, Filtrieren und Waschen erhält man 12,8 g (20 %) der Titelverbindung.
Schmelzpunkt: 180 - 182 °C.
Bruttoformel: $C_9H_{14}N_2O_2S$, Molgewicht: 214,284.

| Elementaranalyse: | | | | |
|---|---|---|---|---|
| berechnet:<br>gemessen: | C=50,45 %;<br>C=50,52 %; | H=6,58 %;<br>H=6,81 %; | N=13,07 %;<br>N=12,85 %; | S=14,96 %;<br>S=14,95 %. |

**Beispiel 32**

Herstellung von Äthyl-(4,6-dimethyl-1,2,3,4-tetra-hydro-2-pyrimidinthion-5-carboxylat)

13,2 g (0,3 Mol) Acetaldehyd, 22,8 g (0,3 Mol) Thioharnstoff und 39 g (0,3 Mol) Acetessigsäureäthylester werden unter den im Beispiel 30 beschriebenen Bedingungen 15 Stunden lang umgesetzt. Nach Kühlen, Filtrieren und Waschen erhält man 30 g (46,7 %) der Titelverbindung.
Schmelzpunkt: 198 - 200 °C.
Bruttoformel: $C_9H_{14}N_2O_2S$, Molgewicht: 214,284.

| Elementaranalyse: | | | | |
|---|---|---|---|---|
| berechnet:<br>gemessen: | C=50,45 %;<br>C=50,52 %; | H=6,54 %;<br>H=6,81 %; | N=13,07 %;<br>N=12,85 %; | S=14,96 %;<br>S=14,95 %. |

**Beispiel 33**

Herstellung von Äthyl-(4-äthyl-6-methyl-1,2,3,4-tetrahydro-2-pyrimidinthion-5-carboxylat)

17,4 g (0,3 Mol) Propionalaldehyd, 22,8 g (0,3 Mol) Thioharnstoff und 39 g (0,3 Mol) Acetessigsäureäthylester werden in 100 ml 15 Gew.-% Chlorwasserstoff enthaltendem Isopropylalkohol (15 g von 100 ml Isopropylalkohol absorbierter gasförmiger Chlorwasserstoff) 35 Stunden lang umgesetzt, dann wird die Lösung unter Vakuum eingedampft, der Rückstand wird aus Wasser kristallisiert, abfiltriert und getrocknet. Man erhält 205 g (30%) der Titelverbindung.
Schmelzpunkt: 143 - 145 °C.
Bruttoformel: $C_{10}H_{16}N_2O_2S$, Molgewicht: 228,312.

| Elementaranalyse: | | | | |
|---|---|---|---|---|
| berechnet:<br>gemessen: | C=50,45 %;<br>C=50,45 %; | H=6,58 %;<br>H=6,58 %; | N=13,07 %;<br>N=13,10 %; | S=14,96 %;<br>S=14,80 %. |

**Beispiel 34**

Herstellung von Äthyl-(4-methyl-6-phenyl-1,2,3,4-tetrahydro-2-pyrimidinthion-5-carboxylat)

13,2 g (0,3 Mol) Acetaldehyd, 22,8 g (0,3 Mol) Thioharnstoff und 57,7 g (0,3 Mol) Benzoylessigsäureäthylester werden unter den im Beispiel 30 beschriebenen Bedingungen 30 Stunden lang umgesetzt. Nach Kühlen, Filtrieren und Waschen erhält man 31,5 g (38 %) der Titelverbindung.
Schmelzpunkt: 220 - 235 °C.
Bruttoformel: $C_{14}H_{16}N_2O_2S$, Molgewicht: 276,356.

| Elementaranalyse: | | | | |
|---|---|---|---|---|
| berechnet:<br>gemessen: | C=52,9 %;<br>C=52,61 %; | H=7,07 %;<br>H=7,06 %; | N=12,50 %;<br>N=12,27 %; | S=14,37 %;<br>S=14,04 %. |

**Beispiel 35**

Herstellung von Äthyl-(4-äthyl-6-phenyl-1,2,3,4--tetrahydro-2-pyrimidinthion-5-carboxylat)

17,4 g (0,3 Mol) Propionaldehyd, 22,8 g (0,3 Mol) Thioharnstoff und 57,7 g (0,3 Mol) Benzoylessigsäureäthylester werden unter den im Beispiel 30 beschriebenen Bedingungen 30 Stunden lang umgesetzt. Nach Kühlen, Filtrieren und Waschen erhält man 30,5 g (35 %) der Titelverbindung.
Schmelzpunkt: 219 - 221 °C.
Bruttoformel: $C_{15}H_{18}N_2O_2S$, Molgewicht: 290,382.

| Elementaranalyse: | | | | |
|---|---|---|---|---|
| berechnet:<br>gemessen: | C=60,85 %;<br>C=60,47 %; | H=5,83 %;<br>H=5,76 %; | N=10,14 %;<br>N=10,11 %; | S=11,60 %;<br>S=11,72 %. |

**Beispiel 36**

Herstellung von 4-Undecyl-6-methyl-1,2,3,4-tetrahydro-2-pyrimidinthion-5-carbonsäureanilid

55,3 g (0,3 Mol) Laurinaldehyd, 228 g (0,3 Mol) Thioharnstoff und 53,2 g (0,3 Mol) Acetessigsäureanilid werden unter den im Beispiel 30 beschriebenen Bedingungen 30 Stunden lang umgesetzt. Nach Kühlen, Filtrieren und Waschen erhält man 29 g (24,1 %) der Titelverbindung.
Schmelzpunkt: 158 - 160 °C.
Bruttoformel: $C_{23}H_{35}N_3OS$, Molgewicht: 401,614.

| Elementaranalyse: | | | | |
|---|---|---|---|---|
| berechnet:<br>gemessen: | C=68,79 %;<br>C=67,46 %; | H=8,78 %;<br>H=8,94 %; | N=10,46 %;<br>N=10s,12 %; | S=7,98 %;<br>S=7,82 %. |

**Ansprüche**

1.) 3,4-Di-(hydro)-2H,6H-pyrimido[2,1-b][1,3]thiazin-derivate der allgemeinen Formel

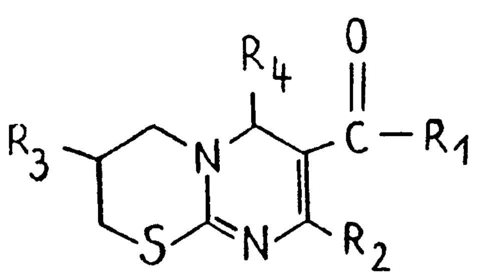

I ,

worin
$R_1$ für einen Alkoxyrest mit 1 bis 6 Kohlenstoffatom(en) oder eine Amino- oder Phenylaminogruppe steht,
$R_2$ einen Alkylrest mit 1 bis 6 Kohlenstoffatom(en) oder einen Phenylrest bedeutet,
$R_3$ ein Wasserstoffatom oder einen Alkylrest mit 1 bis 6 Kohlenstoffatom(en) darstellt und
$R_4$ für einen Alkylrest mit 1 bis 11 Kohlenstoffatom(en) oder einen, gegebenenfalls durch 1 oder mehrere gleiche oder verschiedene Halogenatom(e), Nitrogruppe(n), Aminogruppe(n), Dialkylaminorest(e) mit 1 bis 6 Kohlenstoffatom(en) in jedem Alkylteil, Alkylrest(e) mit 1 bis 6 Kohlenstoffatom(en), Alkoxyrest(e) mit 1 bis 6 Kohlenstoffatom(en) und/oder Hydroxygruppe(n) substituierten, Phenylrest steht,
sowie ihre Säureadditionssalze.

2.) 3,4-Di-(hydro)-2H,6H-pyrimido[2,1-b][1,3]thiazin-derivate nach Anspruch 1, dadurch gekennzeichnet, daß der Alkoxyrest, für den $R_1$ stehen kann, der beziehungsweise die Alkylrest(e), für welche[n] $R_2$ , $R_3$ und/oder $R_4$ stehen kann beziehungsweise können und/oder der beziehungsweise die Alkyl- und/oder Alkoxyrest(e) und/oder die Alkylgruppe(n) des beziehungsweise der Dialkylaminoreste[s], durch welche[n] der Phenylrest, für den $R_4$ stehen kann, substituiert sein kann, [ein] solche[r] mit 1 bis 4, insbesondere 1 oder 2, Kohlenstoffatom(en) ist beziehungsweise sind.

3.) 3,4-Di-(hydro)-2H,6H-pyrimido[2,1-b][1,3]thiazin-derivate nach Anspruch 1 oder 2, dadurch gekennzeichnet, daß das beziehungsweise die Halogenatom(e), durch welche[s] der Phenylrest, für den $R_4$ stehen kann, substituiert sein kann, [ein] Chlor-, Fluor-und/oder Bromatom(e) ist beziehungsweise sind.

4.) Verfahren zur Herstellung der 3,4-Di-(hydro)-2H,6H-pyrimido[2,1-b][1,3]thiazin-derivate nach Anspruch 1 bis 3, dadurch gekennzeichnet, daß man 4,5,6-trisubstituierte 1,2,3,4-Tetra-(hydro)-pyrimidin-2-thione der allgemeinen Formel

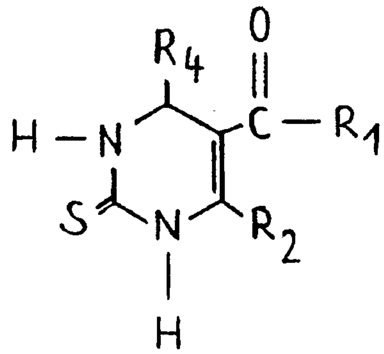

TI ,

worin
$R_1$ , $R_2$
und $R_4$ die in den Ansprüchen 1 bis 3 angegebenen Bedeutungen haben,
mit Dihalogenalkanen der allgemeinen Formel

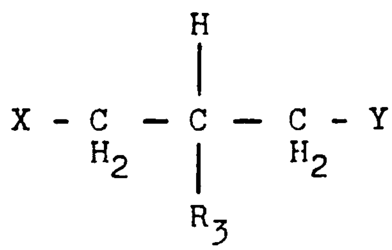

III ,

worin
$R_3$ die im Anspruch 1 oder 2 angegebenen Bedeutungen hat und
X und Y für Halogenatome stehen,
umsetzt, worauf man in an sich bekannter Weise gege benenfalls die erhaltenen 3,4-Di-(hydro)-2H,6H-pyrimido[2,1-b][1,3]thiazin-derivate der allgemeinen Formel I in Säureadditionssalze überführt beziehungsweise gegebenenfalls die erhaltenen Säureadditionssalze der 3,4-Di-(hydro)-2H,6H-pyrimido[2,1-b][1,3] thiazin-derivate der allgemeinen Formel I in die freien 3,4-Di-(hydro)-2H,6H-pyrimido[2,1-b][1,3]thiazin-derivate der allgemeinen Formel I oder in andere Säureadditionssalze überführt.

5.) Verfahren nach Anspruch 4, dadurch gekennzeichnet, daß man die 4,5,6-trisubstituierten 1,2,3,4-Tetra-(hydro)-pyrimidin-2-thione der allgemeinen Formel II und die Dihalogenalkane der allgemeinen Formel III in äquimolaren Mengen verwendet.

6.) Verfahren nach Anspruch 4 oder 5, dadurch gekennzeichnet, daß man die Umsetzung der 4,5,6-trisubstituierten 1,2,3,4-Tetra-(hydro)-pyrimidin-2-thione der allgemeinen Formel II mit den Dihalogenalkanen der allgemeinen Formel III in Gegenwart von 1 oder mehr säurebindenden Mittel(n) und/oder zu ihrer Beschleunigung in Gegenwart von 1 oder mehr Katalysator(en) durchführt.

7.) 4,5,6-Trisubstituierte 1,2,3,4-Tetra-(hydro)-pyrimidin-2-thione der allgemeinen Formel

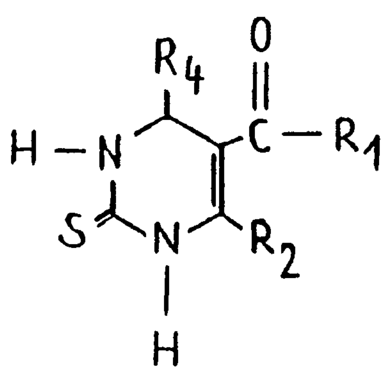

II ,

worin
$R_1$ für einen Alkoxyrest mit 1 bis 6 Kohlenstoffatom(en) oder eine Amino- oder Phenylaminogruppe steht,
$R_2$ einen Alkylrest mit 1 bis 6 Kohlenstoffatom(en) oder einen Phenylrest bedeutet
und
$R_4$ für einen Alkylrest mit 1 bis 11 Kohlenstoffatom(en) steht,
sowie ihre Säureadditionssalze.

8.) Verfahren zur Herstellung der 4,5,6-trisubstituierten 1,2,3,4-Tetra-(hydro)-pyrimidin-2-thione nach Anspruch 7, dadurch gekennzeichnet, daß man Aldehyde der allgemeinen Formel

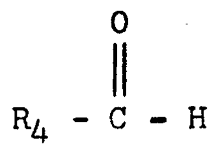

IV ,

worin
$R_4$ die im Anspruch 7 angegebenen Bedeutung hat,
mit $\beta$-Ketocarbonsäurederivaten der allgemeinen Formel

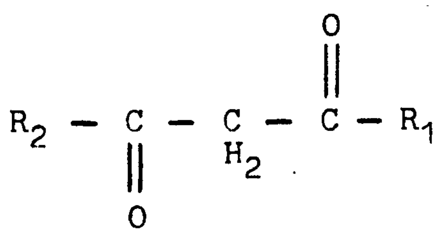

V ,

worin
$R_1$ und $R_2$ die im Anspruch 7 angegebenen Bedeutungen haben,
und mit Thioharnstoff umsetzt, worauf man in an sich bekannter Weise gegebenenfalls die erhaltenen 1,2,3,4-Tetra-(hydro)-pyrimidin-2-thione der allgemeinen Formel II in Säureadditionssalze überführt beziehungsweise gegebenenfalls die erhaltenen Säureadditionssalze der 1,2,3,4-Tetra-(hydro)-pyrimidin-2-thione der allgemeinen Formel II in die freien 1,2,3,4-Tetra-(hydro)-pyrimidin-2-thione der allgemeinen Formel II oder in andere Säureadditionssalze überführt.

9.) Arzneimittel, gekennzeichnet durch einen Gehalt an 1 oder mehr Verbindung(en) nach Anspruch 1 bis 3 und/oder 7, gegebenenfalls zusammen mit 1 oder mehr inerten, festen und/oder flüssigen Träger- und/oder Hilfsstoff(en).

10.) Verwendung der Verbindungen nach Anspruch 1 bis 3 und/oder 7 zur Herstellung von Arzneimitteln, insbesondere mit antianginöser und/oder entzündungshemmender Wirkung.

Patentanspruch für folgenden Vertragsstaat: Gr

1.) Verfahren zur Herstellung von 3,4-Di-(hydro)-2H,6H-pyrimido[2,1-b][1,3]thiazin-derivaten der allgemei-Formel

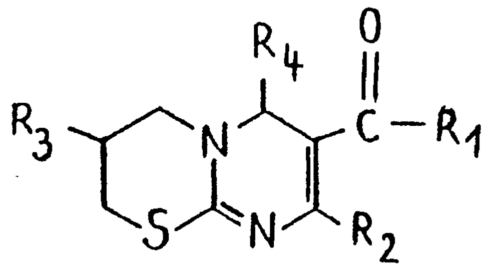

I ,

worin
$R_1$ für einen Alkoxyrest mit 1 bis 6 Kohlenstoffatom(en) oder eine Amino- oder Phenylaminogruppe steht,
$R_2$ einen Alkylrest mit 1 bis 6 Kohlenstoffatom(en) oder einen Phenylrest bedeutet,
$R_3$ ein Wasserstoffatom oder einen Alkylrest mit 1 bis 6 Kohlenstoffatom(en) darstellt
und
$R_4$ für einen Alkylrest mit 1 bis 11 Kohlenstoffatom(en) oder einen, gegebenenfalls durch 1 oder mehrere gleiche oder verschiedene Halogenatom(e), Nitrogruppe(n), Aminogruppe(n), Dialkylaminorest(e) mit 1 bis 6 Kohlenstoffatom(en) in jedem Alkylteil, Alkylrest(e) mit 1 bis 6 Kohlenstoffatom(en), Alkoxyrest(e) mit 1 bis 6 Kohlenstoffatom(en) und/oder Hydroxygruppe(n) substituierten, Phenylrest steht,
sowie ihren Säureadditionssalzen, dadurch gekennzeichnet, daß man 4,5,6-trisubstituierte 1,2,3,4-Tetra-(hydro)-pyrimidin-2-thione der allgemeinen Formel

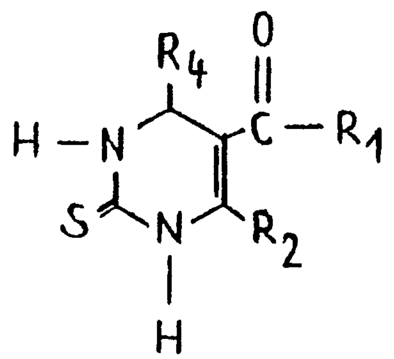

II ,

worin
$R_1$ , $R_2$
und $R_4$ die oben angegebenen Bedeutungen haben,
mit Dihalogenalkanen der allgemeinen Formel

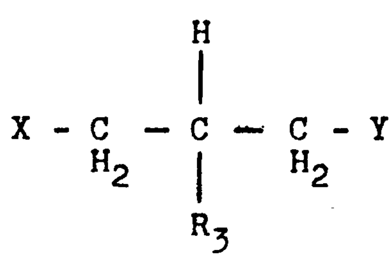

III ,

worin
$R_3$ die oben angegebenen Bedeutungen hat und
X und Y für Halogenatome stehen,
umsetzt, worauf man in an sich bekannter Weise gegebenenfalls die erhaltenen 3,4-Di-(hydro)-2H,6H-pyrimido[2,1-b][1,3]thiazin-derivate der allgemeinen Formel I in Säureadditionssalze überführt beziehungsweise gegebenenfalls die erhaltenen Säureadditionssalze der 3,4-Di-(hydro)-2H,6H-pyrimido[2,1-b][1,3]-thiazin-derivate der allgemeinen Formel I in die freien 3,4-Di-(hydro)-2H,6H-pyrimido[2,1-b][1,3]thiazin-derivate der allgemeinen Formel I oder in andere Säureadditionssalze überführt.

2.) Verfahren nach Anspruch 1, dadurch gekennzeichnet, daß man die 4,5,6-trisubstituierten 1,2,3,4-Tetra-(hydro)-pyrimidin-2-thione der allgemeinen Formel II und die Dihalogenalkane der allgemeinen Formel III in äquimolaren Mengen verwendet.

3.) Verfahren nach Anspruch 1 oder 2, dadurch gekennzeichnet, daß man die Umsetzung der 4,5,6-trisubstituierten 1,2,3,4-Tetra-(hydro)-pyrimidin-2-thione der allgemeinen Formel II mit den Dihalogenalkanen der allgemeinen Formel III in Gegenwart von 1 oder mehr säurebindenden Mittel(n) und/oder zu ihrer Beschleunigung in Gegenwart von 1 oder mehr Katalysator(en) durchführt.

4.) Verfahren nach Anspruch 1 bis 3, dadurch gekennzeichnet, daß man als 3,4-Di-(hydro)-2H,6H-pyrimido-[2,1-b][1,3]thiazin-derivate solche, bei denen der Alkoxyrest, für den $R_1$ stehen kann, der beziehungsweise die Alkylrest(e), für welche[n] $R_2$, $R_3$ und/oder $R_4$ stehen kann beziehungsweise können und/oder der beziehungsweise die Alkyl- und/oder Alkoxyrest(e) und/oder die Alkylgruppe(n) des beziehungsweise der Dialkylaminoreste[s], durch welche[n] der Phenylrest, für den $R_4$ stehen kann, substituiert sein kann, [ein] solche[r] mit 1 bis 4, insbesondere 1 oder 2, Kohlenstoffatom(en) ist beziehungsweise sind, herstellt.

5.) Verfahren nach Anspruch 1 bis 4, dadurch gekennzeichnet, daß man als 3,4-Di-(hydro)-2H,6H-pyrimido-[2,1-b][1,3]thiazin-derivate solche, bei denen das beziehungsweise die Halogenatom(e), durch welche[s] der Phenylrest, für den $R_4$ stehen kann, substituiert sein kann, [ein] Chlor-, Fluor- und/oder Bromatom(e) ist beziehungsweise sind, herstellt.

6.) Verfahren zur Herstellung von 4,5,6-trisubstituierten 1,2,3,4-Tetra-(hydro)-pyrimidin-2-thionen der allgemeinen Formel

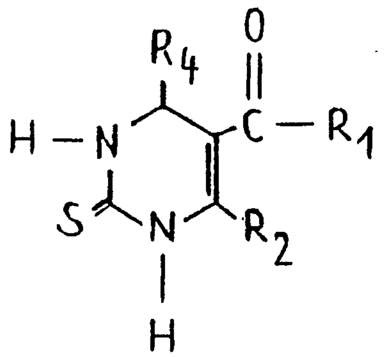

. II ,

worin

$R_1$ für einen Alkoxyrest mit 1 bis 6 Kohlenstoffatom(en) oder eine Amino- oder Phenylaminogruppe steht,

$R_2$ einen Alkylrest mit 1 bis 6 Kohlenstoffatom(en) oder einen Phenylrest bedeutet

und

$R_4$ für einen Alkylrest mit 1 bis 11 Kohlenstoffatom(en) steht,

sowie ihren Säureadditionssalzen, dadurch gekennzeichnet, daß man Aldehyde der allgemeinen Formel

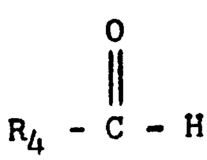

IV ,

worin

$R_4$ die oben angegebene Bedeutung hat,

mit $\beta$-Ketocarbonsäurederivaten der allgemeinen Formel

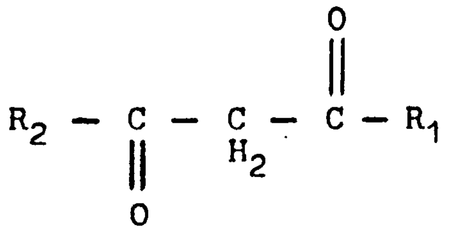

V ,

worin
$R_1$ und $R_2$ die oben angegebenen Bedeutungen haben,
und mit Thioharnstoff umsetzt, worauf man in an sich bekannter Weise gegebenenfalls die erhaltenen 1,2,3,4-Tetra-(hydro)-pyrimidin-2-thione der allgemeinen Formel II in Säureadditionssalze überführt beziehungsweise gegebenenfalls die erhaltenen Säureadditionssalze der 1,2,3,4-Tetra-(hydro)-pyrimidin-2-thione der allgemeinen Formel II in die freien 1,2,3,4-Tetra-(hydro)-pyrimidin-2-thione der allgemeinen Formel II oder in andere Säureadditionssalze überführt.